# EUROPEAN PATENT APPLICATION

(11) **EP 4 589 356 A2**
(43) Date of publication of application: **23.07.2025**
(21) Application number: 24223679.2
(22) Date of filing: 30.12.2024
(51) Int. Cl.: G02B 21/00, G01N 21/64, G02B 21/08, G02B 21/24

(54) **IMAGING SYSTEM AND SEQUENCING SYSTEM**

(30) Priority: 29.12.2023 CN 202311870074; 29.12.2023 CN 202323669472 U
(71) Applicant: GeneMind Biosciences Company Limited, Shenzhen City, 518023 Guangdong (CN)
(72) Inventor: XU, Jiawen, Shenzhen City (CN); LI, Yang, Shenzhen City (CN); LI, Mingze, Shenzhen City (CN); WANG, Guangming, Shenzhen City (CN)
(74) Representative: Schiweck Weinzierl Koch Patentanwälte Partnerschaft mbB

(57) **Abstract**

The present disclosure discloses an imaging system and a sequencing system. The imaging system comprises a first carrier stage, a lens module arranged on the first carrier stage, and a first support seat, a second splitter module, a focusing lens set and a detector module arranged on a benchtop of the first carrier stage. The optical axis of the lens module is perpendicular to the benchtop. The first support seat is provided with a light source module, a focusing module, and a first splitter module. The light source module is arranged aside from the optical axis of the lens module. The focusing module and the first splitter module are located on the optical axis of the lens module, and the first splitter module is located between the lens module and the focusing module. At least part of the second splitter module is located on the optical axis of the lens module and located between the first splitter module and the lens module. The focusing lens set is located between the second splitter module and the detector module. As such, the present disclosure improves the integration of the imaging system and reduces the space occupation of the imaging system in the horizontal direction, making the imaging system compact in structure and reducing the volume of the imaging system.

## Description

### TECHNICAL FIELD

The present disclosure relates to the field of gene sequencing, and in particular, to an imaging system and a sequencing system.

### BACKGROUND

The gene sequencing technology refers to techniques for acquiring the base sequence of DNA or RNA by assays. The current dominant sequencing technique is high-throughput sequencing, in which the general gene sequencing process includes: fixing a nucleic acid sample of interest on a biochip by hybridization; forming a nucleic acid molecule cluster on the nucleic acid sample of interest using PCR amplification; adding bases with a fluorophore, a polymerase, a primer, and the like; bonding the bases with the fluorophore to the base on the nucleic acid sample of interest via the base complementary pairing principle; exciting the fluorophore by using an optical imaging system to generate fluorescence; acquiring the fluorescence for forming an image; and performing base calling on the image, so as to achieve base sequence determination of the nucleic acid sample of interest.

At present, optical imaging systems of gene sequencers have two configurations. In one configuration, the whole optical imaging system is arranged vertically, while in the other one, the whole optical imaging system is arranged horizontally. Typically, the multichannel imaging systems are arranged on the same reference plane, such that the optical imaging system needs more space horizontally.

### SUMMARY

The present disclosure provides an imaging system and a sequencing system.

The imaging system according to embodiments of the present application includes:
a first carrier stage including a benchtop; a lens module arranged on the first carrier stage, where the optical axis of the lens module is perpendicular to the benchtop; a first support seat arranged on the benchtop, where the first support seat is provided with a light source module, a focusing module, and a first splitter module, the light source module is arranged aside from the optical axis of the lens module, the focusing module and the first splitter module are located on the optical axis of the lens module, and the first splitter module is located between the lens module and the focusing module; and a second splitter module, a focusing lens set, and a detector module that are arranged on the benchtop, where at least part of the second splitter module is located on the optical axis of the lens module and located between the first splitter module and the lens module, and the focusing lens set is located between the second splitter module and the detector module.

In the imaging system according to embodiments of the present application, the light source module, the focusing module, and the first splitter module are integrated on the first support seat; the optical axis of the lens module is perpendicular to the benchtop; the second splitter module, the focusing lens set, and the detector module are distributed on the horizontal benchtop. As such, the integration level of the imaging system is improved, and the space occupation of the imaging system in the horizontal direction is reduced, making the imaging system compact in structure and reducing the volume of the imaging system. Also, such a design reduces the distance between the focusing lens set and the detector module, so as to reduce the sizes of the first splitter module and the focusing lens set, thus making the imaging system more compact in structure and further reducing the volume of the imaging system.

In some certain embodiments, an optical correction element is arranged between the lens module and the second splitter module, and the optical correction element can be moved into or out of the optical path of the imaging system; when the optical correction element is moved into the imaging optical path, the optical correction element is located on the optical axis of the lens module.

As such, the optical correction element can correct the aberration between the first surface and the second surface of the sample of interest, such that the imaging system can sharply image the first surface and the second surface of the sample of interest and acquire optical signals on the two surfaces of the sample of interest for imaging, thus improving the sequencing efficiency and the sequencing throughput.

In some certain embodiments, the lens module includes an objective lens, and the center deviation between the optical correction element and the objective lens is not greater than 0.5 mm.

When the center deviation between the optical correction element and the objective lens meets the requirements, the image quality correction effect of the optical correction element can be improved, and therefore the imaging quality of the imaging system on the sample of interest is improved.

In some certain embodiments, the first support seat includes a first surface and a second surface; the first surface of the first support seat is parallel to the optical axis of the lens module, the second surface of the first support seat is perpendicular to the optical axis of the lens module, and the light source module and the focusing module are respectively mounted on the first surface of the first support seat and the second surface of the first support seat.

As such, the light source module and the focusing module can be arranged in the perpendicular direction and the parallel direction relative to the first support seat, thus reducing the space occupation of the imaging system in the horizontal direction, making the imaging system compact in structure, and reducing the volume of the imaging system.

In some certain embodiments, the light source module includes a first light source; the first light source emits a first light beam, and the first light beam is sequentially reflected by the first splitter module and transmitted by the second splitter module to irradiate the sample of interest through the lens module, so as to excite the sample of interest to generate an emission light with at least one wavelength.

As such, the first light beam emitted by the first light source irradiates the sample of interest and excites the optically detectable label on the sample of interest to generate an emission light with at least one wavelength. In some certain embodiments, the focusing module includes a second light source and a focusing sensor; the second light source emits a second light beam, and the second light beam is sequentially transmitted by the first splitter module and the second splitter module to irradiate the sample of interest through the lens module; the focusing sensor detects the second light beam reflected by the surface of the sample of interest.

As such, the focusing module is mainly configured for marking the object distance after the lens module finishes the focusing, monitoring the variation of the object distance in real time during the gene sequencing process, and correcting the variation, so as to ensure that the system always focuses sharply, ensure the accuracy of image acquisition by the detector module, and finally give sharp images within the depth of focus range, while achieving automatic focusing and improving the sequencing efficiency.

In some certain embodiments, the first splitter module includes a first dichroic mirror; the first dichroic mirror is located on the optical axis of the lens module and reflects the first light beam and transmits the second light beam.

As such, the first dichroic mirror reflects the first light beam to the lens module, such that the first light beam irradiates the sample of interest after passing through the lens module to excite the sample of interest to generate a plurality of emission lights with different wavelengths.

In some certain embodiments, the second splitter module includes a second dichroic mirror; the second dichroic mirror is located on the optical axis of the lens module and between the first dichroic mirror and the lens module, receives the first light beam and the second light beam from the first dichroic mirror, and transmits the first light beam and the second light beam to the lens module.

As such, the second dichroic mirror transmits the first light beam to the lens module, such that the first light beam irradiates the sample of interest after passing through the lens module to excite the sample of interest to generate a plurality of emission lights with different wavelengths. In addition, the second dichroic mirror transmits the second light beam to the lens module to adjust the relative positions of the lens module and the sample of interest.

In some certain embodiments, the second splitter module further includes a third dichroic mirror, a fourth dichroic mirror, and a fifth dichroic mirror; the first light beam excites the sample of interest to generate four emission lights with different wavelengths; the lens module acquires the emission lights and transmits the emission lights to the second dichroic mirror; the second dichroic mirror reflects the emission lights to the third dichroic mirror; the third dichroic mirror splits the emission lights from the second dichroic mirror into a first mixed light beam and a second mixed light beam, reflects the first mixed light beam to the fourth dichroic mirror, and transmits the second mixed light beam to the fifth dichroic mirror; the fourth dichroic mirror transmits a portion of the first mixed light beam to form a third light beam and reflects another portion of the first mixed light beam to form a fourth light beam; the fifth dichroic mirror transmits a portion of the second mixed light beam to form a fifth light beam and reflects another portion of the second mixed light beam to form a sixth light beam.

As such, the third dichroic mirror, the fourth dichroic mirror, and the fifth dichroic mirror split a plurality of emission lights with different wavelengths from the lens module by wavelength to form a plurality of light beams, so as to achieve the detection of the plurality of emission lights with different wavelengths emitted by the sample of interest.

In some certain embodiments, the imaging system includes a second support seat mounted on the benchtop, and the second dichroic mirror, the third dichroic mirror, the fourth dichroic mirror, and the fifth dichroic mirror are all integrally arranged on the second support seat.

Such a design facilitates the adjustment of the second dichroic mirror, the third dichroic mirror, the fourth dichroic mirror, and the fifth dichroic mirror, reduces the distances between the second dichroic mirror, the third dichroic mirror, the fourth dichroic mirror, and the fifth dichroic mirror, and reduces the sizes of the second dichroic mirror, the third dichroic mirror, the fourth dichroic mirror, and the fifth dichroic mirror, thereby making the imaging system more compact in structure and improving the imaging quality.

In some certain embodiments, when the optical correction element is moved into the imaging optical path, the optical correction element is located between the lens module and the second dichroic mirror and on the optical axis of the lens module.

As such, the optical correction element can compensate for the astigmatism caused by the imaging of the light beam from the lens module, and improve the imaging effect of the image formed by the light beam received by the detector module through the optical correction element.

In some certain embodiments, the detector module includes a first image sensor, a second image sensor, a third image sensor, and a fourth image sensor that are arranged on the benchtop; the first image sensor is configured for receiving the third light beam and forming a first image; the second image sensor is configured for receiving the fourth light beam and forming a second image; the third image sensor is configured for receiving the fifth light beam and forming a third image; the fourth image sensor is configured for receiving the sixth light beam and forming a fourth image.

As such, the four image sensors are adopted to receive different light beams and form images at different spatial positions, so as to make the imaging system compact in structure, reduce the volume of the imaging system, and improve the space utilization rate, while achieving the simultaneous acquisition of the fluorescence signals corresponding to base groups and improving the sequencing efficiency and the sequencing throughput.

In some certain embodiments, the focusing lens set includes a first tube lens, a second tube lens, a third tube lens, and a fourth tube lens; the first tube lens is arranged between the fourth dichroic mirror and the first image sensor and is configured for converging the third light beam onto the first image sensor; the second tube lens is arranged between the fourth dichroic mirror and the second image sensor and is configured for converging the fourth light beam onto the second image sensor; the third tube lens is arranged between the fifth dichroic mirror and the third image sensor and is configured for converging the fifth light beam onto the third image sensor; the fourth tube lens is arranged between the fifth dichroic mirror and the fourth image sensor and is configured for converging the sixth light beam onto the fourth image sensor.

As such, the focusing lens set can cooperate with the lens module to converge the light beams to the detector module for imaging.

In some certain embodiments, the imaging system includes a mirror set mounted on the benchtop; the mirror set including a first mirror, a second mirror, a third mirror, and a fourth mirror; the first mirror is arranged between the first tube lens and the first image sensor and is configured for reflecting the third light beam from the first tube lens to the first image sensor; the second mirror is arranged between the second tube lens and the second image sensor and is configured for reflecting the fourth light beam from the second tube lens to the second image sensor; the third mirror is arranged between the third tube lens and the third image sensor and is configured for reflecting the fifth light beam from the third tube lens to the third image sensor; the fourth mirror is arranged between the fourth tube lens and the fourth image sensor and is configured for reflecting the sixth light beam from the fourth tube lens to the fourth image sensor.

As such, the direction of the light beam can be changed through the mirror set, such that the first image sensor, the second image sensor, the third image sensor, and the fourth image sensor can be distributed at different positions on the benchtop, thus making the imaging system compact in structure, reducing the volume of the imaging system, and improving the space utilization rate.

In some certain embodiments, the light source module further includes a base, a lens tube holder, a first optical assembly, and a second optical assembly; the lens tube holder is arranged on the base; the first optical assembly is arranged on the lens tube holder; the second optical assembly is arranged on the lens tube holder, located on a light emergent side of the first optical assembly, and capable of rotating around the optical axis of the second optical assembly and moving along the optical axis of the second optical assembly relative to the lens tube holder to adjust the size, the shape, and/or the emergent direction of the spot of the first light beam.

As such, the second optical assembly can rotate around the optical axis of the second optical assembly and move along the optical axis of the second optical assembly relative to the lens tube holder, thus eliminating the need for mirrors to facilitate the movement and rotation of the second optical assembly, making the imaging system compact in structure, and improving the adjustment effect.

In some certain embodiments, the first optical assembly includes an adjusting seat and a first lens set arranged in the adjusting seat, and the adjusting seat is capable of rotating around the optical axis of the first optical assembly and moving along the optical axis of the first optical assembly relative to the lens tube holder.

As such, the first lens set can rotate around the optical axis of the first optical assembly and move along the optical axis of the first optical assembly relative to the lens tube holder, thus facilitating the adjustment of the size, the shape, and the emergent direction of the spot of the first light beam.

In some certain embodiments, the first lens set includes a collimating lens, and the collimating lens is configured for collimating light.

As such, the collimating lens can collimate the first light beam into a parallel light beam to keep the first light beam parallel in the transmission process, reducing the energy loss and the light beam diffusion.

In some certain embodiments, the first optical assembly further includes a first mounting seat mounted in the adjusting seat, and the first lens set is mounted in the first mounting seat.

As such, the first lens set can be arranged in the adjusting seat through the first mounting seat, which facilitates the adjustment of the first lens set. Also, the first mounting seat can protect the first lens set, reduce the risk of damage to the first lens set, and improve the service life of the imaging system.

In some certain embodiments, the first mounting seat is in a threaded connection with the adjusting seat. As such, the threaded connection makes it convenient to assemble and disassemble the first mounting seat and the adjusting seat, facilitating the adjustment of the first mounting seat.

In some certain embodiments, the first optical assembly includes an optical fiber adapter plate arranged on the optical axis of the first lens set, and the optical fiber adapter plate is located on the side of the first lens set distal to the second optical assembly.

As such, the first light beam can be output through the optical fiber adapter plate to excite the sample of interest to generate an emission light with at least one wavelength.

In some certain embodiments, the light source module includes a filter element arranged in the lens tube holder, and the filter element is located between the first optical assembly and the second optical assembly.

As such, the filter element can filter the first light beam, transmit the first light beam with a specific wavelength, and cut off lights with other wavelengths, thereby improving the imaging quality of the imaging system.

In some certain embodiments, the lens tube holder is provided with a first locking mechanism, and the first locking mechanism is configured for forming a fitting connection with the adjusting seat to lock the first optical assembly on the lens tube holder.

As such, the first optical assembly can be fixed on the lens tube holder through the first locking mechanism, thus avoiding the movement of the first optical assembly relative to the lens tube holder and ensuring the stability of the imaging system.

In some certain embodiments, the first locking mechanism includes a first locking member, and the first locking member is arranged through the lens tube holder and locks the adjusting seat when the first locking member abuts against the adjusting seat.

As such, the adjusting seat can be fixed on the lens tube holder through the first locking member, thus avoiding the movement of the adjusting seat relative to the lens tube holder and ensuring the stability of the imaging system.

In some certain embodiments, the first locking member includes a first threaded member, and the lens tube holder is provided with a first threaded hole extending in the radial direction of the lens tube holder; the first threaded member is partially screwed in the first threaded hole.

As such, the adjusting seat can be fixed on the lens tube holder through the first threaded member, thus avoiding the movement of the adjusting seat relative to the lens tube holder and ensuring the stability of the imaging system. In addition, the first threaded member makes it convenient to disassemble the adjusting seat from the lens tube holder, facilitating the adjustment of the adjusting seat.

In some certain embodiments, the second optical assembly includes a second mounting seat and a second lens set; the second lens set is mounted in the second mounting seat, and the second mounting seat is capable of rotating around the optical axis of the second optical assembly and moving along the optical axis of the second optical assembly relative to the lens tube holder.

As such, the second lens set can rotate around the optical axis of the second optical assembly and move along the optical axis of the second optical assembly relative to the lens tube holder, thus facilitating the adjustment of the size, the shape, and the emergent direction of the spot of the first light beam.

In some certain embodiments, the second lens set includes at least two cylindrical lenses.

As such, the cylindrical lens can shape the first light beam such that the spot formed by the irradiation of the first light beam on the sample of interest can just cover the imaging region.

In some certain embodiments, the lens tube holder is provided with a second locking mechanism, and the second locking mechanism is configured for forming a fitting connection with the second mounting seat to lock the second optical assembly on the lens tube holder.

As such, the second optical assembly can be fixed on the lens tube holder through the second locking mechanism, thus avoiding the movement of the second optical assembly relative to the lens tube holder and ensuring the stability of the imaging system.

In some certain embodiments, the lens tube holder is formed with a first slot and a second slot; the first slot passes through the tube wall of the lens tube holder and extends to an end surface of the lens tube holder in the axial direction of the lens tube holder, the second slot passes through the tube wall of the lens tube holder and extends in the circumferential direction of the lens tube holder, and one end of the second slot is communicated with the first slot; the second locking mechanism includes a second locking member arranged on the lens tube holder, and the second locking member is configured for adjusting the size of the first slot to adjust the inner diameter of the lens tube holder, such that the lens tube holder holds the second mounting seat tightly to lock the second optical assembly on the lens tube holder.

As such, the second optical assembly can be fixed on the lens tube holder through the second locking member, thus avoiding the movement of the second optical assembly relative to the lens tube holder and ensuring the stability of the imaging system.

In some certain embodiments, the second locking member includes a second threaded member, and the lens tube holder is provided with a second threaded hole extending in a tangential direction of the lens tube holder; the second threaded member is partially screwed in the second threaded hole.

As such, the second mounting seat can be fixed on the lens tube holder through the second threaded member, thus avoiding the movement of the second mounting seat relative to the lens tube holder and ensuring the stability of the imaging system. In addition, the second threaded member makes it convenient to disassemble the second mounting seat from the lens tube holder, facilitating the adjustment of the second mounting seat. In some certain embodiments, the imaging system further includes an adjusting mechanism arranged on the base, the lens tube holder is connected with the adjusting mechanism, and the adjusting mechanism is configured for adjusting the position of the lens tube holder relative to the base in the radial direction of the lens tube holder and adjusting the pitch angle of the lens tube holder relative to the base.

As such, the adjustment of the pitch angle of the lens tube holder relative to the base allows the spot formed by the irradiation of the first light beam through the lens module on the sample of interest to coincide with the imaging region through the lens module.

In some certain embodiments, the adjusting mechanism includes a connecting member and a first adjusting member arranged on the connecting member; the connecting member is mounted on the base, and the first adjusting member is connected with the lens tube holder.

As such, the lens tube holder can be driven by the first adjusting member to move in a direction perpendicular to the optical axis of the first optical assembly relative to the connecting member, so as to adjust the height of the lens tube holder.

In some certain embodiments, the first adjusting member includes a third threaded member, and the lens tube holder is provided with a third threaded hole extending in the radial direction of the lens tube holder; the third threaded member is arranged through the connecting member and partially screwed in the third threaded hole. As such, the lens tube holder can be driven by rotating the third threaded member to move in a direction perpendicular to the optical axis of the first optical assembly relative to the connecting member, so as to adjust the height of the lens tube holder, featuring a simple structure, ease to operate, and improved adjustment precision of the first adjusting member.

In some certain embodiments, the adjusting mechanism includes a second adjusting member arranged on the connecting member, and the second adjusting member is connected with the base and configured for adjusting the position of the lens tube holder relative to the base to adjust the pitch angle of the lens tube holder relative to the base.

As such, the connecting member can be driven by the second adjusting member to perform pitch motion relative to the base, so as to drive the lens tube holder to perform pitch adjustment relative to the base.

In some certain embodiments, the second adjusting member includes a fourth threaded member, and the connecting member is provided with a fourth threaded hole extending in the axial direction of the lens tube holder; the fourth threaded member is arranged through the connecting member and abuts against the base.

As such, the connecting member can be driven by rotating the fourth threaded member to perform pitch motion relative to the base, so as to drive the lens tube holder to perform pitch adjustment relative to the base, featuring a simple structure, ease to operate, and improved adjustment precision of the second adjusting member.

In some certain embodiments, the connecting member is provided with a third locking mechanism, and the third locking mechanism is configured for forming a fitting connection with the base to lock the connecting member on the base, so as to keep the lens tube holder stable relative to the base.

As such, the connecting member can be fixed on the base through the third locking mechanism, thus avoiding the movement of the connecting member relative to the base and ensuring the stability of the imaging system. In some certain embodiments, the third locking mechanism includes a third locking member; the third locking member is arranged through the connecting member and locks the connecting member when the third locking member is connected with the base.

As such, the connecting member can be fixed on the base through the third locking mechanism, thus avoiding the movement of the connecting member relative to the base and ensuring the stability of the imaging system. In some certain embodiments, the third locking member includes a fifth threaded member, and the base is provided with a fifth threaded hole extending in the axial direction of the lens tube holder; the fifth threaded member is partially screwed in the fifth threaded hole.

As such, the connecting member can be fixed on the base through the fifth threaded member, thus avoiding the movement of the connecting member relative to the base and ensuring the stability of the imaging system. In addition, the first threaded member makes it convenient to disassemble the connecting member from the base, facilitating the adjustment of the connecting member.

The sequencing system according to embodiments of the present application includes the imaging system.

The sequencing system according to embodiments of the present application possesses a compact structure and a high sequencing efficiency.

In some certain embodiments, the sequencing system includes a mounting bracket and a second carrier stage; the second carrier stage is arranged on the mounting bracket, located in the optical axis extending direction of the lens module and perpendicular to the optical axis of the lens module, and configured for carrying the sample of interest.

As such, the sample of interest can be placed on the second carrier stage, which facilitates the imaging of the sample of interest by the imaging system.

In some certain embodiments, the mounting bracket is formed with an accommodating space, and the second carrier stage is accommodated in the accommodating space.

As such, the second carrier stage can be accommodated in the mounting bracket through the accommodating space, which facilitates the imaging of the sample of interest by the imaging system.

In some certain embodiments, the sequencing system includes a movable platform mechanism, the movable platform mechanism is accommodated in the accommodating space, the second carrier stage is mounted on the movable platform mechanism, and the movable platform mechanism can drive the second carrier stage to move.

As such, the movable platform mechanism drives the sample of interest to move through driving the second carrier stage to move, which facilitates the imaging of the different regions of the sample of interest by the imaging system.

In some certain embodiments, the mounting bracket is provided with a vibration damping structure configured for reducing the vibration of the sequencing system.

As such, the vibration damping structure can reduce the vibration of the sequencing system, and thus further improve the detection accuracy of the sequencing system.

Additional aspects and advantages of the present disclosure will in part be illustrated in the following description and become apparent from the following description, or may be learned by the implementation of the present disclosure.

### BRIEF DESCRIPTION OF THE DRAWINGS

The aforementioned and/or additional aspects and advantages of the present disclosure will become apparent and easily understood from the description of the embodiments with reference to the following drawings, in which:
FIG. 1 illustrates a schematic of the imaging system according to embodiments of the present disclosure;
FIG. 2 illustrates a structural schematic of the sequencing system according to embodiments of the present disclosure;
FIG. 3 illustrates a structural schematic of the sequencing system according to embodiments of the present disclosure;
FIG. 4 illustrates a structural schematic of the sequencing system according to embodiments of the present disclosure;
FIG. 5 illustrates a structural schematic of the first support seat according to embodiments of the present disclosure;
FIG. 6 illustrates a structural schematic of the second splitter module according to embodiments of the present disclosure;
FIG. 7 illustrates a structural schematic of the light source module according to embodiments of the present disclosure;
FIG. 8 illustrates a structural schematic of the light source module according to embodiments of the present disclosure;
FIG. 9 illustrates a cross-sectional schematic taken in direction A-A of FIG. 8;
FIG. 10 illustrates a structural schematic of the light source module according to embodiments of the present disclosure;
FIG. 11 illustrates a structural schematic of the light source module according to embodiments of the present disclosure; and
FIG. 12 illustrates a structural schematic of the light source module according to embodiments of the present disclosure.

Description of reference numerals: 100, imaging system; 10, first carrier stage; 11, first support seat; 12, benchtop; 13, first surface; 14, second surface; 15, second support seat; 20, lens module; 21, objective lens; 30, second splitter module; 31, second dichroic mirror; 32, third dichroic mirror; 33, fourth dichroic mirror; 34, fifth dichroic mirror; 40, focusing lens set; 41, first tube lens; 42, second tube lens; 43, third tube lens; 44, fourth tube lens; 45, first filter; 46, second filter; 47, third filter; 48, fourth filter; 50, detector module; 51, first image sensor; 52, second image sensor; 53, third image sensor; 54, fourth image sensor; 60, light source module; 61, first light source; 62, first light beam; 63, lens; 64, first mixed light beam; 65, second mixed light beam; 66, third light beam; 67, fourth light beam; 68, fifth light beam; 69, sixth light beam; 70, focusing module; 71, second light source; 72, focusing sensor; 73, second light beam; 80, first splitter module; 81, first dichroic mirror; 90, optical correction element; 91, mirror set; 92, first mirror; 93, second mirror; 94, third mirror; 95, fourth mirror; 110, base; 111, through hole; 120, lens tube holder; 121, first locking mechanism; 122, first locking member; 123, first threaded member; 124, first threaded hole; 125, second threaded hole; 130, first optical assembly; 131, adjusting seat; 132, first lens set; 133, collimating lens; 134, first mounting seat; 135, retaining ring; 136, optical fiber adapter plate; 140, second optical assembly; 141, second mounting seat; 142, second lens set; 143, cylindrical lens; 150, filter element; 161, first slot; 162, second slot; 170, adjusting mechanism; 171, connecting member; 172, first adjusting member; 173, third threaded member; 174, third threaded hole; 175, second adjusting member; 176, fourth threaded member; 177, fourth threaded hole; 178, third locking mechanism; 179, third locking member; 180, fifth threaded member; 181, fifth threaded hole; 182, elastic member; 200, sequencing system; 210, mounting bracket; 211, accommodating space; 220, second carrier stage; 230, movable platform mechanism; 240, vibration damping structure; 300, sample of interest.

### DETAILED DESCRIPTION

The embodiments of the present disclosure are described in detail below, and the examples of the embodiments are shown in the accompanying drawings, throughout which identical or similar reference numerals represent identical or similar elements or elements having identical or similar functionality. The embodiments described below with reference to the drawings are exemplary and are merely intended to illustrate the present disclosure, but should be construed as limiting the present disclosure.

In the description of the present disclosure, it will be appreciated that orientational or positional relationships indicated by terms such as "central", "longitudinal", "transverse", "length", "width", "thickness", "upper", "lower", "front", "rear", "left", "right", "vertical", "horizontal", "top", "bottom", "inner", "outer", "clockwise", "counterclockwise", and the like are those shown on the basis of the accompanying drawings, and are merely intended to facilitate and simplify the description rather than indicate or imply that the indicated device or element must have a specific orientation and be configured and operated according to the specific orientation. Such relationships should not be construed as limiting the present disclosure. In addition, the terms "first" and "second" are used herein for descriptive purposes only and should not be construed as indicating or implying relative importance or implicitly indicating the number of technical features described. Therefore, features defined with "first" and "second" may explicitly or implicitly include one or more of the features. In the description of the present disclosure, unless otherwise specifically defined, the "plurality" means two or more.

In the description of the present disclosure, it should be noted that unless otherwise clearly specified and defined, the terms "mount", "link", and "connect" should be interpreted in their broad sense. For example, the connection may be a fixed connection, detachable connection, or integral connection; a mechanic connection, electric connection, or communicative connection; or a direct connection, indirect connection through an intermediate, internal communication of two elements, or interaction between two elements. For those of ordinary skill in the art, the specific meanings of the aforementioned terms in the present disclosure can be interpreted according to specific conditions.

In the present disclosure, unless explicitly stated or limited otherwise, a first feature being "above" or "below" a second feature may encompass that the first and second features are in direct contact, and that the first and second features are not in direct contact but are in contact via an additional feature between them. Also, a first feature being "on", "over", and "above" a second feature encompasses that the first feature is right above or obliquely above the second feature, or simply means that the first feature is at a vertically higher position than the second feature. A first feature being "under", "beneath", and "below" a second feature encompasses that the first feature is right below or obliquely below the second feature, or simply means that the first feature is at a vertically lower position than the second feature.

The following disclosure provides many different embodiments or examples for implementing different structures of the present disclosure. To simplify the disclosure of the present application, the components and settings of specific examples are described below. Certainly, the examples are merely exemplary and are not intended to limit the present disclosure. In addition, reference numerals and/or characters may be repeatedly used in different examples in the present disclosure for simplicity and clarity rather than for indicating the relationship between various embodiments and/or settings discussed. In addition, the present disclosure provides examples of various specific processes and materials, but those of ordinary skill in the art will recognize the application of other processes and/or the use of other materials.

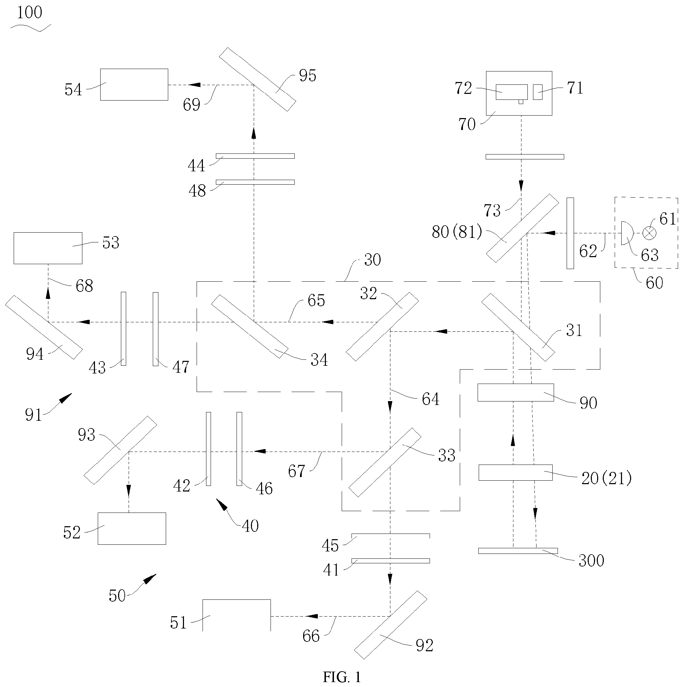

Referring to FIGs. 1 to 4, the imaging system 100 according to embodiments of the present application includes a first carrier stage 10, a lens module 20, a first support seat 11, a second splitter module 30, a focusing lens set 40, and a detector module 50, where the first carrier stage 10 includes a benchtop 12; the lens module 20 is arranged on the first carrier stage 10, and the optical axis of the lens module 20 is perpendicular to the benchtop 12; the first support seat 11, the second splitter module 30, the focusing lens set 40, and the detector module 50 are arranged on the benchtop 12; the first support seat 11 is provided with a light source module 60, a focusing module 70, and a first splitter module 80; the light source module 60 is arranged aside from the optical axis of the lens module 20; the focusing module 70 and the first splitter module 80 are located on the optical axis of the lens module 20, and the first splitter module 80 is located between the lens module 20 and the focusing module 70; at least part of the second splitter module 30 is located on the optical axis of the lens module 20 and between the first splitter module 80 and the lens module 20; the focusing lens set 40 is located between the second splitter module 30 and the detector module 50.

In the imaging system 100 according to embodiments of the present application, the light source module 60, the focusing module 70, and the first splitter module 80 are integrated on the first support seat 11; the optical axis of the lens module 20 is perpendicular to the benchtop 12; the second splitter module 30, the focusing lens set 40, and the detector module 50 are distributed on the horizontal benchtop 12. As such, the integration level of the imaging system 100 is improved, and the space occupation of the imaging system 100 in the horizontal direction is reduced, making the imaging system 100 compact in structure and reducing the volume of the imaging system 100. Also, such a design reduces the distance between the focusing lens set 40 and the detector module 50, so as to reduce the sizes of the first splitter module 80 and the focusing lens set 40, thus making the imaging system 100 more compact in structure and further reducing the volume of the imaging system 100.

Specifically, the first carrier stage 10 may have a square plate structure, and the four corners of the first carrier stage 10 may be designed into rounded corners, so as to prevent the sharp corners from injuring the operator. The lens module 20 is mounted on the first carrier stage 10 and is capable of moving in the optical axis of the lens module 20 relative to the first carrier stage 10. The first support seat 11, the second splitter module 30, the focusing lens set 40, and the detector module 50 may be fixed on the first carrier stage 10 by fasteners such as bolts.

The lens module 20 is configured for receiving a plurality of emission lights with different wavelengths generated by the sample of interest 300 after being excited by the excitation light, and transmitting the emission lights to the second splitter module 30.

The first support seat 11 may be formed by two opposite side plates and a flat plate. The side plates and the flat plate have a rectangle plate structure. The flat plate is located on the tops of the two side plates, the two side plates are perpendicular to the benchtop 12, and the bottom surfaces of the side plates extend outward, such that the two side plates can be fixed on the benchtop 12 of the first carrier stage 10 through fasteners such as bolts. The two side plates are connected to the flat plate and are fixed through fasteners such as bolts. The second splitter module 30 can split the emission lights from the lens module 20 into two beams, three beams, four beams, etc., and direct the beams to the detector module 50, so as to achieve the detection of the emission lights with different wavelengths emitted by the sample of interest 300.

The focusing lens set 40 is configured for cooperating with the lens module 20 to converge the light beams to the detector module 50 for imaging, where the focusing lens set 40 may be a tube lens, or the focusing lens set 40 may include one or more tube lenses.

The detector module 50 is configured for acquiring the optical signals acquired by the lens module 20 and forming an image, where the detector module 50 may be an image sensor, or the detector module 50 may include one or more image sensors.

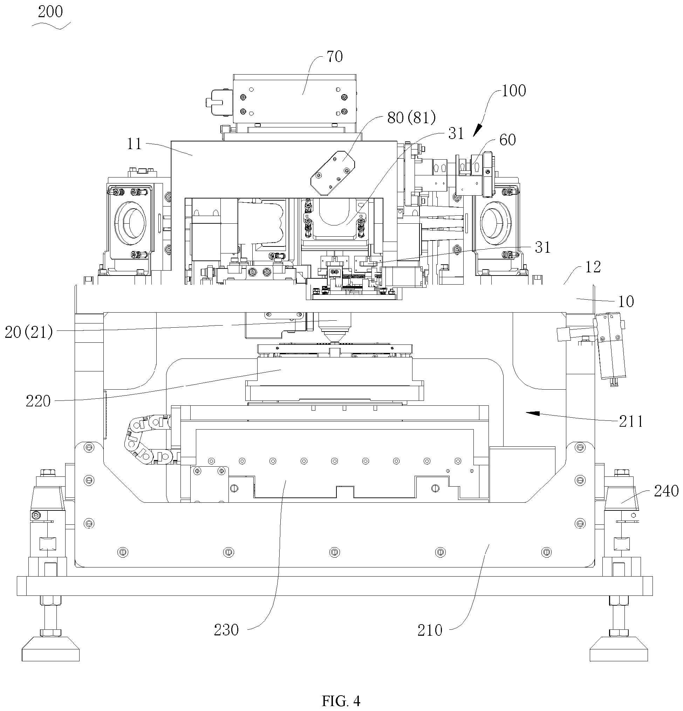

Referring to FIGs. 1 and 4, in some certain embodiments, an optical correction element 90 is arranged between the lens module 20 and the second splitter module 30, and the optical correction element 90 can be moved into or out of the optical path of the imaging system 100; when the optical correction element 90 is moved into the imaging optical path, the optical correction element 90 is located on the optical axis of the lens module 20.

As such, the optical correction element 90 can correct the aberration between the two surfaces of the sample of interest 300, such that the imaging system 100 can sharply image the two surfaces of the sample of interest 300 and acquire optical signals on the two surfaces of the sample of interest 300 for imaging, thus improving the sequencing efficiency and the sequencing throughput.

Specifically, the light incident surface and/or the light emergent surface of the optical correction element 90 are perpendicular to the optical axis of the lens module 20.

In some certain embodiments, the lens module 20 includes an objective lens 21, and the center deviation between the optical correction element 90 and the objective lens 21 is not greater than 0.5 mm.

When the center deviation between the optical correction element 90 and the objective lens 21 meets the requirements, the image quality correction effect of the optical correction element 90 can be improved, and therefore the imaging quality of the imaging system 100 on the sample of interest 300 is improved.

Specifically, the objective lens 21 may be an available objective lens 21, such as an infinity conjugate objective lens 21, or the objective lens 21 may be a customized objective lens 21. The focal length of the objective lens 21 can be set according to the requirements of the imaging system 100. The center deviation between the optical correction element 90 and the objective lens 21 refers to the distance between the optical axis of the optical correction element 90 and the optical axis of the objective lens 21, and a smaller center deviation between the optical correction element 90 and the objective lens 21 will result in a higher imaging quality of the imaging system 100 for the sample of interest 300.

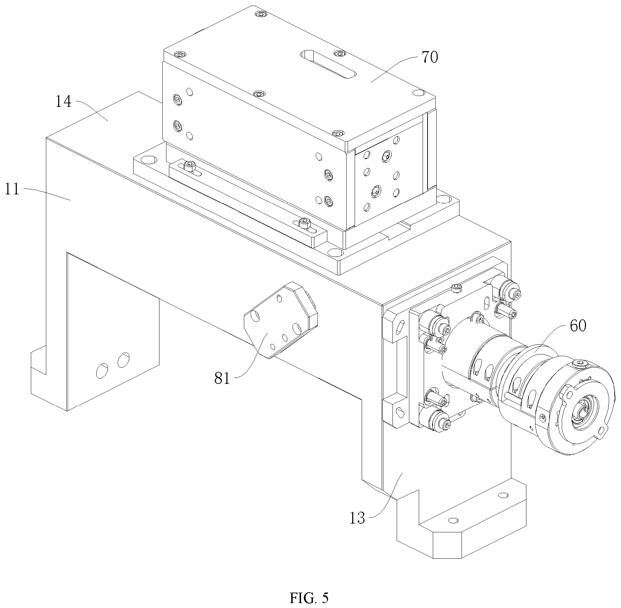

Referring to FIGs. 1 and 5, in some certain embodiments, the first support seat 11 includes a first surface 13 and a second surface 14; the first surface 13 of the first support seat 11 is parallel to the optical axis of the lens module 20, the second surface 14 of the first support seat 11 is perpendicular to the optical axis of the lens module 20, and the light source module 60 and the focusing module 70 are respectively mounted on the first surface 13 of the first support seat 11 and the second surface 14 of the first support seat 11.

As such, the light source module 60 and the focusing module 70 can be arranged in the perpendicular direction and the parallel direction relative to the first support seat 11, thus reducing the space occupation of the imaging system 100 in the horizontal direction, making the imaging system 100 compact in structure, and reducing the volume of the imaging system 100.

Specifically, the first surface 13 of the first support seat 11 is perpendicular to the benchtop 12, and the light source module 60 is mounted on the first surface 13 of the first support seat 11; the second surface 14 of the first support seat 11 is parallel to the benchtop 12, and the focusing module 70 is mounted on the second surface 14 of the first support seat 11.

Referring to FIG. 1, in some certain embodiments, the light source module 60 includes a first light source 61; the first light source 61 emits a first light beam 62, and the first light beam 62 is sequentially reflected by the first splitter module 80 and transmitted by the second splitter module 30 to irradiate the sample of interest 300 through the lens module 20, so as to excite the sample of interest 300 to generate an emission light with at least one wavelength.

As such, the first light beam 62 emitted by the first light source 61 irradiates the sample of interest 300 and excites the optically detectable label on the sample of interest 300 to generate an emission light with at least one wavelength.

Specifically, a lens 63 may be arranged on the illumination optical path of the first light source 61. By adjusting the distance between the lens 63 and the lens module 20, the beam aperture of the first light beam 62 entering the lens module 20 is controlled, such that the first light beam 62 completely enters the lens module 20 and is projected to the sample of interest 300 through the lens module 20, so as to excite the sample of interest 300 to generate the emission light. In some embodiments, the lens 63 is an aspherical lens.

Referring to FIG. 1, in some certain embodiments, the focusing module 70 includes a second light source 71 and a focusing sensor 72; the second light source 71 emits a second light beam 73, and the second light beam 73 is sequentially transmitted by the first splitter module 80 and the second splitter module 30 to irradiate the sample of interest 300 through the lens module 20; the focusing sensor 72 detects the second light beam 73 reflected by the surface of the sample of interest 300.

As such, the focusing module 70 is mainly configured for marking the object distance after the lens module 20 finishes the focusing, monitoring the variation of the object distance in real time during the gene sequencing process, and correcting the variation, so as to ensure that the system always focuses sharply, ensure the accuracy of image acquisition by the detector module 50, and finally give sharp images within the depth of focus range, while achieving automatic focusing and improving the sequencing efficiency.

Specifically, the second light beam 73 emitted by the second light source 71 is sequentially transmitted by the first dichroic mirror 81, the second dichroic mirror 31, the optical correction element 90, and the lens module 20 to irradiate the sample of interest 300 and returns along the original path. The focusing sensor 72 is configured for receiving a light beam reflected by the sample of interest 300 and collimated by the lens module 20. The focusing sensor 72 determines a distance between the plane where the sample of interest 300 is located and a focal plane of the lens module 20 according to the light beam, and converts an optical signal into an electrical signal. The electrical signal is configured for characterizing the distance between the plane where the sample of interest 300 is located and the focal plane of the lens module 20, and the lens module 20 is driven to move along its optical axis according to the electrical signal, so as to achieve automatic focusing. Referring to FIG. 1, in some certain embodiments, the first splitter module 80 includes a first dichroic mirror 81; the first dichroic mirror 81 is located on the optical axis of the lens module 20 and reflects the first light beam 62 and transmits the second light beam 73.

As such, the first dichroic mirror 81 reflects the first light beam 62 to the lens module 20, such that the first light beam 62 irradiates the sample of interest 300 after passing through the lens module 20 to excite the sample of interest 300 to generate a plurality of emission lights with different wavelengths.

In some embodiments, the first dichroic mirror 81 is positioned at an included angle of 45° with respect to the optical axis of the lens module 20. The lens module 20 and the first light source 61 are located on the same side of the first dichroic mirror 81, such that the first light source 61 is incident on the lens module 20. Referring to FIG. 1, in some certain embodiments, the second splitter module 30 includes a second dichroic mirror 31; the second dichroic mirror 31 is located on the optical axis of the lens module 20 and between the first dichroic mirror 81 and the lens module 20, receives the first light beam 62 and the second light beam 73 from the first dichroic mirror 81, and transmits the first light beam 62 and the second light beam 73 to the lens module 20.

As such, the second dichroic mirror 31 transmits the first light beam 62 to the lens module 20, such that the first light beam 62 irradiates the sample of interest 300 after passing through the lens module 20 to excite the sample of interest 300 to generate a plurality of emission lights with different wavelengths. In addition, the second dichroic mirror 31 transmits the second light beam 73 to the lens module 20 to adjust the relative positions of the lens module 20 and the sample of interest 300.

In some embodiments, the second dichroic mirror 31 is arranged at an included angle of 45° with respect to the optical axis of the lens module 20. The second dichroic mirror 31 may form an included angle of 90° with respect to the first dichroic mirror 81, or the second dichroic mirror 31 may be parallel to the first dichroic mirror 81. The second dichroic mirror 31 may reflect the plurality of emission lights with different wavelengths from lens module 20 and transmit the second light beam 73 from lens module 20 to the focusing module 70.

Referring to FIG. 1, in some certain embodiments, the second splitter module 30 further includes a third dichroic mirror 32, a fourth dichroic mirror 33, and a fifth dichroic mirror 34; the first light beam 62 excites the sample of interest 300 to generate four emission lights with different wavelengths; the lens module 20 acquires the emission lights and transmits the emission lights to the second dichroic mirror 31; the second dichroic mirror 31 reflects the emission lights to the third dichroic mirror 32; the third dichroic mirror 32 splits the emission lights from the second dichroic mirror 31 into a first mixed light beam 64 and a second mixed light beam 65, reflects the first mixed light beam 64 to the fourth dichroic mirror 33, and transmits the second mixed light beam 65 to the fifth dichroic mirror 34; the fourth dichroic mirror 33 transmits a portion of the first mixed light beam 64 to form a third light beam 66 and reflects another portion of the first mixed light beam 64 to form a fourth light beam 67; the fifth dichroic mirror 34 transmits a portion of the second mixed light beam 65 to form a fifth light beam 68 and reflects another portion of the second mixed light beam 65 to form a sixth light beam 69.

As such, the third dichroic mirror 32, the fourth dichroic mirror 33, and the fifth dichroic mirror 34 split a plurality of emission lights with different wavelengths from the lens module 20 by wavelength to form a plurality of light beams, so as to achieve the detection of the plurality of emission lights with different wavelengths emitted by the sample of interest 300.

Specifically, the third dichroic mirror 32 may be arranged at an included angle of 90° with respect to the second dichroic mirror 31. The fourth dichroic mirror 33 may be arranged at an included angle of 90° with respect to the second dichroic mirror 31. The fifth dichroic mirror 34 may be arranged in parallel with respect to the second dichroic mirror 31.

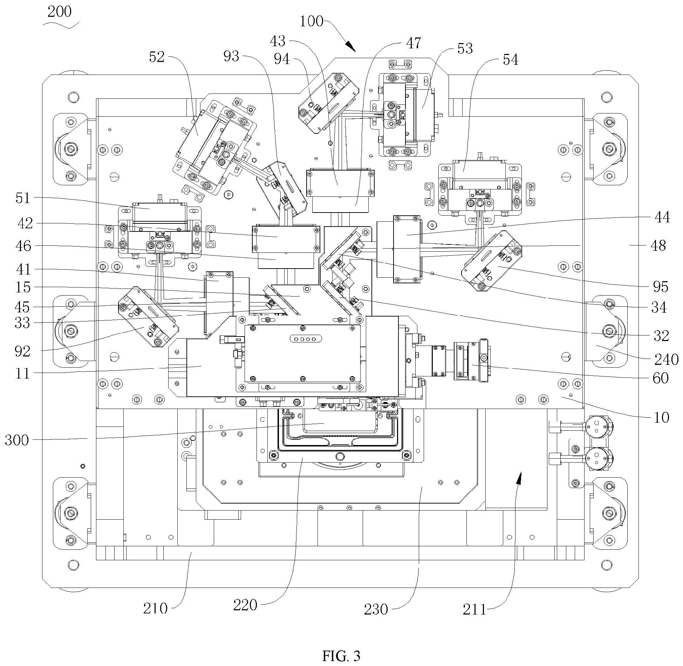

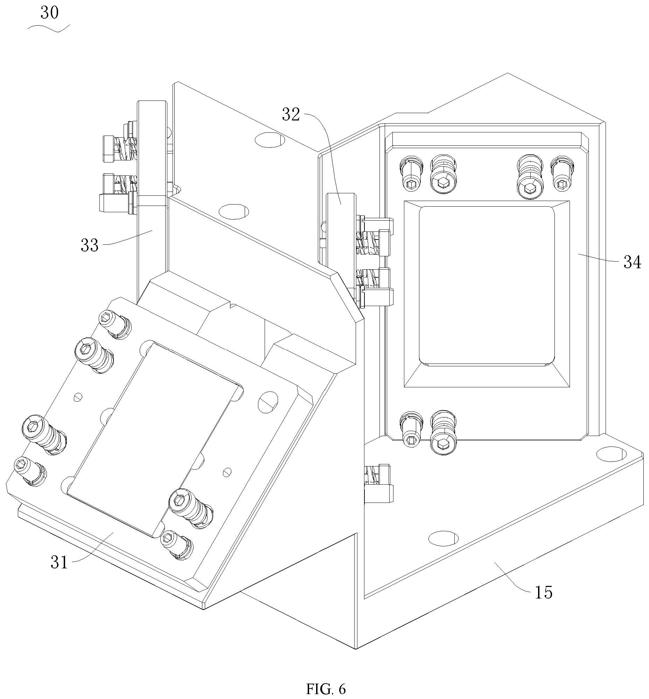

Referring to FIGs. 3 and 6, in some certain embodiments, the imaging system 100 includes a second support seat 15 mounted on the benchtop 12. The second dichroic mirror 31, the third dichroic mirror 32, the fourth dichroic mirror 33, and the fifth dichroic mirror 34 are all integrally arranged on the second support seat 15. Such a design facilitates the adjustment of the second dichroic mirror 31, the third dichroic mirror 32, the fourth dichroic mirror 33, and the fifth dichroic mirror 34, reduces the distances between the second dichroic mirror 31, the third dichroic mirror 32, the fourth dichroic mirror 33, and the fifth dichroic mirror 34, and reduces the sizes of the second dichroic mirror 31, the third dichroic mirror 32, the fourth dichroic mirror 33, and the fifth dichroic mirror 34, thereby making the imaging system 100 more compact in structure and improving the imaging quality.

Specifically, the second support seat 15 may be fixed to the benchtop 12 through fasteners such as bolts. The second dichroic mirror 31, the third dichroic mirror 32, the fourth dichroic mirror 33, and the fifth dichroic mirror 34 may be fixed to the second support seat 15 through fasteners such as bolts. The second support seat 15 may consist of a plurality of triangular prism structures and a flat plate structure, or may be integrally formed.

Referring to FIGs. 1 and 4, in some certain embodiments, when the optical correction element 90 is moved into the imaging optical path, the optical correction element 90 is located between the lens module 20 and the second dichroic mirror 31 and on the optical axis of the lens module 20.

As such, the optical correction element 90 can compensate for the astigmatism caused by the imaging of the light beam from the lens module 20, and improve the imaging effect of the image formed by the light beam received by the detector module 50 through the optical correction element 90.

Referring to FIGs. 1 and 3, in some certain embodiments, the detector module 50 includes four cameras arranged on the benchtop 12; the four cameras include a first image sensor 51, a second image sensor 52, a third image sensor 53, and a fourth image sensor 54, respectively; the first image sensor 51 is configured for receiving the third light beam 66 and forming a first image; the second image sensor 52 is configured for receiving the fourth light beam 67 and forming a second image; the third image sensor 53 is configured for receiving the fifth light beam 68 and forming a third image; the fourth image sensor 54 is configured for receiving the sixth light beam 69 and forming a fourth image.

As such, the four image sensors are adopted to receive different light beams and form images at different spatial positions, so as to make the imaging system 100 compact in structure, reduce the volume of the imaging system 100, and improve the space utilization rate, while achieving the simultaneous acquisition of the fluorescence signals corresponding to base groups and improving the sequencing efficiency and the sequencing throughput.

Specifically, the first image sensor 51, the second image sensor 52, the third image sensor 53, and the fourth image sensor 54 may be any sensors capable of sensing optical image information and converting the same into usable output signals, including but not limited to CCD and CMOS.

Referring to FIG. 1, in some certain embodiments, the focusing lens set 40 includes a first tube lens 41, a second tube lens 42, a third tube lens 43, and a fourth tube lens 44; the first tube lens 41 is arranged between the fourth dichroic mirror 33 and the first image sensor 51 and is configured for converging the third light beam 66 onto the first image sensor 51; the second tube lens 42 is arranged between the fourth dichroic mirror 33 and the second image sensor 52 and is configured for converging the fourth light beam 67 onto the second image sensor 52; the third tube lens 43 is arranged between the fifth dichroic mirror 34 and the third image sensor 53 and is configured for converging the fifth light beam 68 onto the third image sensor 53; the fourth tube lens 44 is arranged between the fifth dichroic mirror 34 and the fourth image sensor 54 and is configured for converging the sixth light beam 69 onto the fourth image sensor 54.

As such, the focusing lens set 40 can cooperate with the lens module 20 to converge the light beams to the detector module 50 for imaging.

Specifically, the first tube lens 41 is arranged on the object side of the first image sensor 51; the second tube lens 42 is arranged on the object side of the second image sensor 52; the third tube lens 43 is arranged on the object side of the third image sensor 53; the fourth tube lens 44 is arranged on the object side of the fourth image sensor 54. The first tube lens 41, the second tube lens 42, the third tube lens 43, and the fourth tube lens 44 may be tube lenses with the same focal length, or may be designed as tube lenses with different focal lengths according to the imaging requirements.

With reference to FIGs. 1 and 3, in some embodiments, the imaging system 100 includes a first filter 45, a second filter 46, a third filter 47, and a fourth filter 48 that are arranged on the benchtop 12; the first filter 45 is arranged on the side of the first tube lens 41 distal to the first image sensor 51 and is configured for filtering the third light beam 66 entering the first tube lens 41; the second filter 46 is arranged on the side of the second tube lens 42 distal to the second image sensor 52 and is configured for filtering the fourth light beam 67 entering the second tube lens 42; the third filter 47 is arranged on the side of the third tube lens 43 distal to the third image sensor 53 and is configured for filtering the fifth light beam 68 entering the third tube lens 43; the fourth filter 48 is arranged on the side of the fourth tube lens 44 distal to the fourth image sensor 54 and is configured for filtering the sixth light beam 69 entering the fourth tube lens 44. Each of the filters transmits an emission light with a specific wavelength, and cuts off emission lights or stray lights with other wavelengths.

Referring to FIGs. 1 and 3, in some certain embodiments, the imaging system 100 includes a mirror set 91 mounted on the benchtop 12; the mirror set 91 including a first mirror 92, a second mirror 93, a third mirror 94, and a fourth mirror 95; the first mirror 92 is arranged between the first tube lens 41 and the first image sensor 51 and is configured for reflecting the third light beam 66 from the first tube lens 41 to the first image sensor 51; the second mirror 93 is arranged between the second tube lens 42 and the second image sensor 52 and is configured for reflecting the fourth light beam 67 from the second tube lens 42 to the second image sensor 52; the third mirror 94 is arranged between the third tube lens 43 and the third image sensor 53 and is configured for reflecting the fifth light beam 68 from the third tube lens 43 to the third image sensor 53; the fourth mirror 95 is arranged between the fourth tube lens 44 and the fourth image sensor 54 and is configured for reflecting the sixth light beam 69 from the fourth tube lens 44 to the fourth image sensor 54.

As such, the direction of the light beam can be changed through the mirror set 91, such that the first image sensor 51, the second image sensor 52, the third image sensor 53, and the fourth image sensor 54 can be distributed at different positions on the benchtop 12, thus making the imaging system 100 compact in structure, reducing the volume of the imaging system 100, and improving the space utilization rate.

Specifically, the first mirror 92 is arranged on the object side of the first image sensor 51; the second mirror 93 is arranged on the object side of the second image sensor 52; the third mirror 94 is arranged on the object side of the third image sensor 53; the fourth mirror 95 is arranged on the object side of the fourth image sensor 54. The first mirror 92, the second mirror 93, the third mirror 94, and the fourth mirror 95 may be fixed on the first carrier stage 10 through fasteners such as bolts.

In one example, the imaging process of the imaging system 100 is as follows: when the first light source 61 emits the first light beam 62, the first light beam 62 is reflected by the first dichroic mirror 81 and transmitted by the second dichroic mirror 31 to irradiate the sample of interest 300 so as to excite the sample of interest 300 to generate an emission light with at least one wavelength; the emission light is acquired by the lens module 20, reflected by the second dichroic mirror 31, and split by the third dichroic mirror 32 to form the first mixed light beam 64 and the second mixed light beam 65; the first mixed light beam 64 is split by the fourth dichroic mirror 33 to form the third light beam 66 and the fourth light beam 67; the second mixed light beam 65 is split by the fifth dichroic mirror 34 to form the fifth light beam 68 and the sixth light beam 69; the first tube lens 41 converges the third light beam 66 to the first mirror 92; the first mirror 92 reflects the third light beam 66 to the first image sensor 51 for imaging; the second tube lens 42 converges the fourth light beam 67 to the second mirror 93; the second mirror 93 reflects the fourth light beam 67 to the second image sensor 52 for imaging; the third tube lens 43 converges the fifth light beam 68 to the third mirror 94; the third mirror 94 reflects the fifth light beam 68 to the third image sensor 53 for imaging; the fourth tube lens 44 converges the sixth light beam 69 to the fourth mirror 95; the fourth mirror 95 reflects the sixth light beam 69 to the fourth image sensor 54 for imaging.

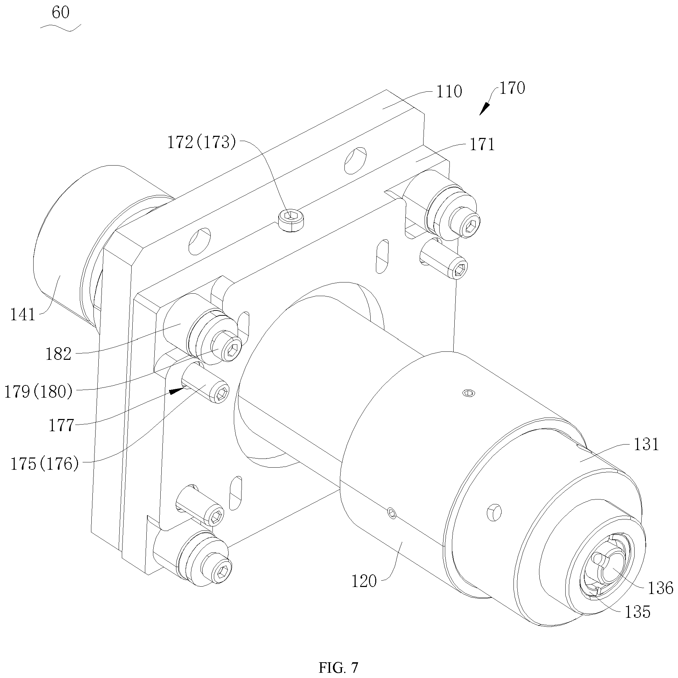

Referring to FIGs. 7 to 9, in some certain embodiments, the light source module 60 further includes a base 110, a lens tube holder 120, a first optical assembly 130, and a second optical assembly 140; the lens tube holder 120 is arranged on the base 110; the first optical assembly 130 is arranged on the lens tube holder 120; the second optical assembly 140 is arranged on the lens tube holder 120, located on a light emergent side of the first optical assembly 130, and capable of rotating around the optical axis of the second optical assembly 140 and moving along the optical axis of the second optical assembly 140 relative to the lens tube holder 120 to adjust the size, the shape, and/or the emergent direction of the spot of the first light beam 62.

As such, the second optical assembly 140 can rotate around the optical axis of the second optical assembly 140 and move along the optical axis of the second optical assembly 140 relative to the lens tube holder 120, thus eliminating the need for mirrors to facilitate the movement and rotation of the second optical assembly 140, making the imaging system 100 compact in structure, and improving the adjustment effect.

Specifically, the base 110 is configured for fixing the lens tube holder 120. The base 110 may have a rectangular plate structure or a circular plate structure. The base 110 is formed with a through hole 111. The through hole 111 passes through the base 110 in the thickness direction of the base 110. The lens tube holder 120 may be arranged through the through hole 111. The thickness direction of the base 110 is consistent with the axial direction of the lens tube holder 120. The lens tube holder 120 may have a circular cylindrical structure with openings at both ends. The first optical assembly 130 and the second optical assembly 140 are arranged at the two ends of the lens tube holder 120 through the openings. By adjusting the distance between the second optical assembly 140 and the first optical assembly 130, the size and the shape of the spot of the first light beam 62 can be adjusted to form an elongated spot that coincides with the imaging region of the detector module 50. In some embodiments, the elongated spot is rectangular.

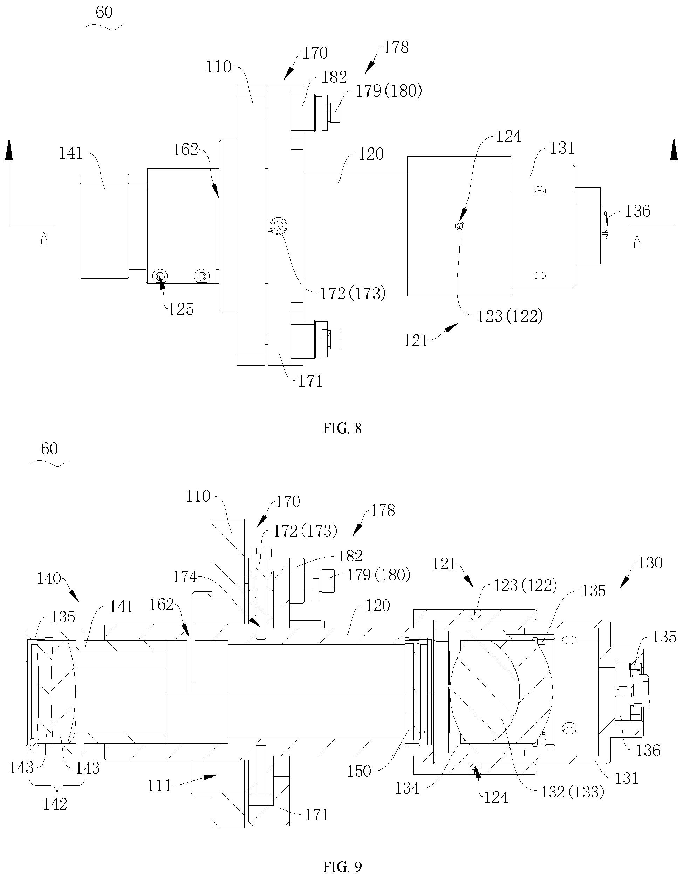

Referring to FIG. 9, in some certain embodiments, the first optical assembly 130 includes an adjusting seat 131 and a first lens set 132 arranged in the adjusting seat 131, and the adjusting seat 131 is capable of rotating around the optical axis of the first optical assembly 130 and moving along the optical axis of the first optical assembly 130 relative to the lens tube holder 120.

As such, the first lens set 132 can rotate around the optical axis of the first optical assembly 130 and move along the optical axis of the first optical assembly 130 relative to the lens tube holder 120, thus facilitating the adjustment of the size, the shape, and the emergent direction of the spot of the first light beam 62.

Specifically, the adjusting seat 131 may be cylindrical. The diameter of the adjusting seat 131 is smaller than that of the lens tube holder 120. The adjusting seat 131 is in clearance fit with an axial hole of the lens tube holder 120, such that the adjusting seat 131 can rotate around and move along the optical axis of the first optical assembly 130 relative to the lens tube holder 120.

Referring to FIG. 9, in some certain embodiments, the first lens set 132 includes a collimating lens 133, and the collimating lens 133 is configured for collimating light.

As such, the collimating lens 133 can collimate the first light beam 62 into a parallel light beam to keep the first light beam 62 parallel in the transmission process, reducing the energy loss and the light beam diffusion. Specifically, the collimating lens 133 may be a spherical lens or an aspherical lens, and the collimating lens 133 may be one lens or a lens set formed by a plurality of lenses.

Referring to FIG. 9, in some certain embodiments, the first optical assembly 130 further includes a first mounting seat 134 mounted in the adjusting seat 131, and the first lens set 132 is mounted in the first mounting seat 134.

As such, the first lens set 132 can be arranged in the adjusting seat 131 through the first mounting seat 134, which facilitates the adjustment of the first lens set 132. Also, the first mounting seat 134 can protect the first lens set 132, reduce the risk of damage to the first lens set 132, and improve the service life of the imaging system 100.

Specifically, the first mounting seat 134 may be cylindrical. The diameter of the first mounting seat 134 is smaller than that of the adjusting seat 131. The first lens set 132 is fixed to the end of the first mounting seat 134 proximal to the second optical assembly 140 through a retaining ring 135. The first light beam 62 can be collimated by adjusting the position of the first mounting seat 134 relative to the adjusting seat 131.

In some certain embodiments, the first mounting seat 134 is in a threaded connection with the adjusting seat 131.

As such, the threaded connection makes it convenient to assemble and disassemble the first mounting seat 134 and the adjusting seat 131, facilitating the adjustment of the first mounting seat 134.

Specifically, the thread may be a triangular thread, a rectangular thread, a trapezoidal thread, a zigzag thread, and the like. The adjusting seat 131 may be formed with a plurality of threaded holes. By partially screwing a fastener such as a bolt or a screw in the threaded hole until one end of the fastener abuts against the first mounting seat 134, the first mounting seat 134 is detachably and fixedly connected with the adjusting seat 131.

Referring to FIG. 9, in some certain embodiments, the first optical assembly 130 includes an optical fiber adapter plate 136 arranged on the optical axis of the first lens set 132, and the optical fiber adapter plate 136 is located on the side of the first lens set 132 distal to the second optical assembly 140.

As such, the first light beam 62 can be output through the optical fiber adapter plate 136 to excite the sample of interest 300 to generate an emission light with at least one wavelength.

Specifically, the optical fiber adapter plate 136 is configured for mounting an optical fiber configured for directing the excitation light. The excitation light is input at the input end of the optical fiber, and the first light beam 62 is output from the output end. The optical fiber adapter plate 136 is arranged in the adjusting seat 131 and located on the side of the adjusting seat 131 distal to the first lens set 132, such that the first lens set 132 collimates the first light beam 62 from the optical fiber adapter plate. In some embodiments, the cross-section of the optical fiber is rectangular, and the first light beam 62 is a rectangular light beam.

Referring to FIG. 9, in some certain embodiments, the light source module 60 includes a filter element 150 arranged in the lens tube holder 120, and the filter element 150 is located between the first optical assembly 130 and the second optical assembly 140.

As such, the filter element 150 can filter the first light beam 62, transmit the first light beam 62 with a specific wavelength, and cut off lights with other wavelengths, thereby improving the imaging quality of the imaging system 100.

Specifically, the filter element 150 filters the first light beam 62 from the first optical assembly 130, and transmits the filtered light beam to the second optical assembly 140. The filter element 150 may be a bandpass filter, a cutoff filter, a splitting filter, and the like. The filter can be selected according to actual requirements. Referring to FIGs. 8 and 9, in some certain embodiments, the lens tube holder 120 is provided with a first locking mechanism 121, and the first locking mechanism 121 is configured for forming a fitting connection with the adjusting seat 131 to lock the first optical assembly 130 on the lens tube holder 120.

As such, the first optical assembly 130 can be fixed on the lens tube holder 120 through the first locking mechanism 121, thus avoiding the movement of the first optical assembly 130 relative to the lens tube holder 120 and ensuring the stability of the imaging system 100.

Referring to FIGs. 8 and 9, in some certain embodiments, the first locking mechanism 121 includes a first locking member 122, and the first locking member 122 is arranged through the lens tube holder 120 and locks the adjusting seat 131 when the first locking member 122 abuts against the adjusting seat 131.

As such, the adjusting seat 131 can be fixed on the lens tube holder 120 through the first locking member 122, thus avoiding the movement of the adjusting seat 131 relative to the lens tube holder 120 and ensuring the stability of the imaging system 100.

Referring to FIGs. 8 and 9, in some certain embodiments, the first locking member 122 includes a first threaded member 123, and the lens tube holder 120 is provided with a first threaded hole 124 extending in the radial direction of the lens tube holder 120; the first threaded member 123 is partially screwed in the first threaded hole 124.

As such, the adjusting seat 131 can be fixed on the lens tube holder 120 through the first threaded member 123, thus avoiding the movement of the adjusting seat 131 relative to the lens tube holder 120 and ensuring the stability of the imaging system 100. In addition, the first threaded member 123 makes it convenient to disassemble the adjusting seat 131 from the lens tube holder 120, facilitating the adjustment of the adjusting seat 131.

Specifically, the numbers of the first threaded members 123 and the first threaded holes 124 may be four, and the four threaded holes are uniformly arranged in the circumferential direction of the lens tube holder 120. The first threaded member 123 includes, but is not limited to, a set screw, a metric screw, a stop screw, and the like. The first threaded hole 124 may pass through the lens tube holder 120 in the radial direction of the lens tube holder 120, and one end of the first threaded member 123 abuts against the adjusting seat 131. By partially screwing the first threaded member 123 in the first threaded hole 124, the adjusting seat 131 and the lens tube holder 120 are detachably and fixedly connected.

Referring to FIGs. 8 and 9, in some certain embodiments, the second optical assembly 140 includes a second mounting seat 141 and a second lens set 142; the second lens set 142 is mounted in the second mounting seat 141, and the second mounting seat 141 is capable of rotating around the optical axis of the second optical assembly 140 and moving along the optical axis of the second optical assembly 140 relative to the lens tube holder 120.

As such, the second lens set 142 can rotate around the optical axis of the second optical assembly 140 and move along the optical axis of the second optical assembly 140 relative to the lens tube holder 120, thus facilitating the adjustment of the size, the shape, and the emergent direction of the spot of the first light beam 62.

Specifically, the second mounting seat 141 may be cylindrical. The second lens set 142 is fixed to the end of the second mounting seat 141 distal to the first optical assembly 130 through a retaining ring 135. The diameter of the end of the second mounting seat 141 proximal to the first optical assembly 130 is smaller than the diameter of the lens tube holder 120. The second mounting seat 141 is in clearance fit with the axial hole of the lens tube holder 120, such that the second mounting seat 141 can rotate around and move along the optical axis of the second optical assembly 140 relative to the lens tube holder 120.

Referring to FIG. 9, in some certain embodiments, the second lens set 142 includes at least two cylindrical lenses 143.

As such, the cylindrical lens 143 can shape the first light beam 62 such that the spot formed by the irradiation of the first light beam 62 on the sample of interest 300 can just cover the imaging region.

Specifically, the cylindrical lens 143 may be an aspherical lens. The cylindrical lens 143 may be cylindrical or semi-cylindrical. The number of the cylindrical lenses 143 may be two, three, four, and the like. The plurality of cylindrical lenses 143 are arranged along the optical axis of the second optical assembly 140. In some embodiments, the number of the cylindrical lenses is two. One cylindrical lens can adjust the collimated first light beam 62 in the length direction, and the other cylindrical lens can adjust the collimated first light beam 62 in the width direction, such that the first light beam 62 forms an elongated light.

In some certain embodiments, the lens tube holder 120 is provided with a second locking mechanism, and the second locking mechanism is configured for forming a fitting connection with the second mounting seat 141 to lock the second optical assembly 140 on the lens tube holder 120.

As such, the second optical assembly 140 can be fixed on the lens tube holder 120 through the second locking mechanism, thus avoiding the movement of the second optical assembly 140 relative to the lens tube holder 120 and ensuring the stability of the imaging system 100.

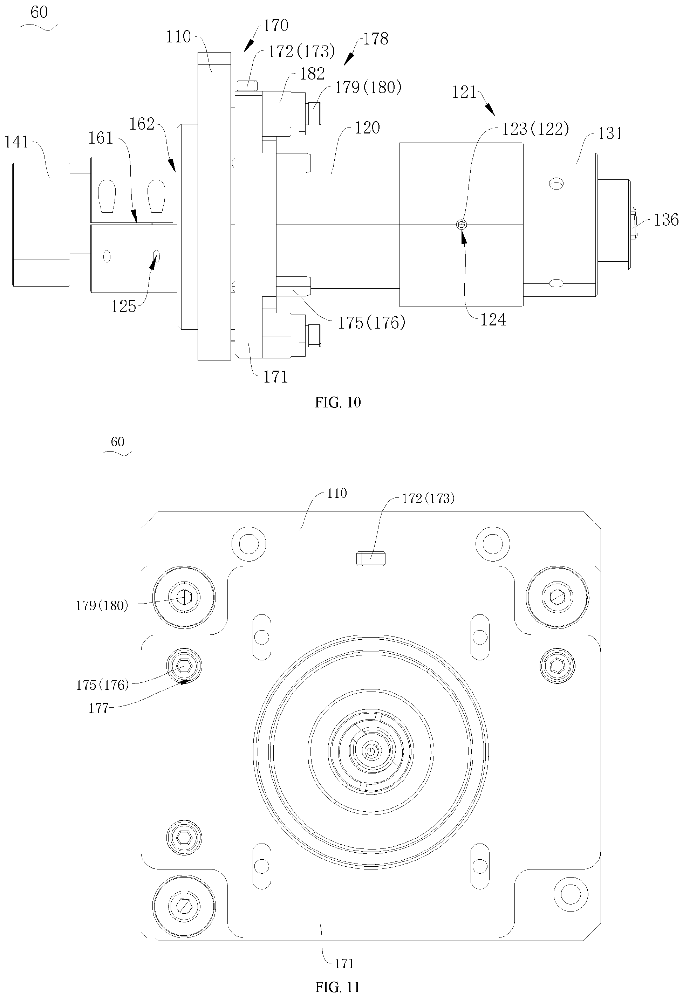

Referring to FIG. 10, in some certain embodiments, the lens tube holder 120 is formed with a first slot 161 and a second slot 162; the first slot 161 passes through the tube wall of the lens tube holder 120 and extends to an end surface of the lens tube holder 120 in the axial direction of the lens tube holder 120, the second slot 162 passes through the tube wall of the lens tube holder 120 and extends in the circumferential direction of the lens tube holder 120, and one end of the second slot 162 is communicated with the first slot 161; the second locking mechanism includes a second locking member arranged on the lens tube holder 120, and the second locking member is configured for adjusting the size of the first slot 161 to adjust the inner diameter of the lens tube holder 120, such that the lens tube holder 120 holds the second mounting seat 141 tightly to lock the second optical assembly 140 on the lens tube holder 120.

As such, the second optical assembly 140 can be fixed on the lens tube holder 120 through the second locking member, thus avoiding the movement of the second optical assembly 140 relative to the lens tube holder 120 and ensuring the stability of the imaging system 100.

Specifically, the first slot 161 is located on the side of the lens tube holder 120 proximal to the second optical assembly 140, and the second slot 162 is partially located in the through hole 111. The length of the first slot 161 may be greater than the length of the second mounting seat 141 arranged in the lens tube holder 120, and the length of the second slot 162 may be one-half of the circumference of the lens tube holder 120. When the width of the first slot 161 is increased, the inner diameter of the lens tube holder 120 is increased. The width of the second slot 162 can be adjusted according to the diameter of the second mounting seat 141, such that the lens tube holder 120 holds the second mounting seat 141 tightly.

Referring to FIG. 10, in some certain embodiments, the second locking member includes a second threaded member, and the lens tube holder 120 is provided with a second threaded hole 125 extending in a tangential direction of the lens tube holder 120; the second threaded member is partially screwed in the second threaded hole 125.

As such, the second mounting seat 141 can be fixed on the lens tube holder 120 through the second threaded member, thus avoiding the movement of the second mounting seat 141 relative to the lens tube holder 120 and ensuring the stability of the imaging system 100. In addition, the second threaded member makes it convenient to disassemble the second mounting seat 141 from the lens tube holder 120, facilitating the adjustment of the second mounting seat 141.

Specifically, the numbers of the second threaded members and the second threaded holes 125 may be two, and the two threaded holes are arranged in the axial direction of the lens tube holder 120. The second threaded member includes, but is not limited to, a set screw, a metric screw, a stop screw, and the like. The second threaded holes 125 may pass through the tube wall of the lens tube holder 120 on both sides of the first slot 161 in the tangential direction of the lens tube holder 120. The second threaded members are connected to the tube wall of the lens tube holder 120 on both sides of the first slot 161. By partially screwing the second threaded member in the second threaded hole 125, the second mounting seat 141 and the lens tube holder 120 are detachably and fixedly connected.

Referring to FIG. 10, in some certain embodiments, the imaging system 100 further includes an adjusting mechanism 170 arranged on the base 110, the lens tube holder 120 is connected with the adjusting mechanism 170, and the adjusting mechanism 170 is configured for adjusting the position of the lens tube holder 120 relative to the base 110 in the radial direction of the lens tube holder 120 and adjusting the pitch angle of the lens tube holder 120 relative to the base 110.

As such, the adjustment of the pitch angle of the lens tube holder 120 relative to the base 110 allows the spot formed by the irradiation of the first light beam 62 through the lens module 20 on the sample of interest 300 to coincide with the imaging region through the lens module 20.

Specifically, the lens tube holder 120 can move in the radial direction of the lens tube holder 120 relative to the base 110 to adjust the position of the lens tube holder 120 relative to the base 110 in the radial direction of the lens tube holder 120, and the lens tube holder 120 can also rotate around the axis of the lens tube holder 120 relative to the base 110 to adjust the pitch angle of the lens tube holder 120 relative to the base 110.

Referring to FIG. 10, in some certain embodiments, the adjusting mechanism 170 includes a connecting member 171 and a first adjusting member 172 arranged on the connecting member 171; the connecting member 171 is mounted on the base 110, and the first adjusting member 172 is connected with the lens tube holder 120.

As such, the lens tube holder 120 can be driven by the first adjusting member 172 to move in a direction perpendicular to the optical axis of the first optical assembly 130 relative to the connecting member 171, so as to adjust the height of the lens tube holder 120.

Specifically, the connecting member 171 may be a plate structure; the connecting member 171 is detachably connected to the base 110 and can rotate around the axis of the lens tube holder 120 relative to the base 110. Referring to FIG. 9, in some certain embodiments, the first adjusting member 172 includes a third threaded member 173, and the lens tube holder 120 is provided with a third threaded hole 174 extending in the radial direction of the lens tube holder 120; the third threaded member 173 is arranged through the connecting member 171 and partially screwed in the third threaded hole 174.

As such, the lens tube holder 120 can be driven by rotating the third threaded member 173 to move in a direction perpendicular to the optical axis of the first optical assembly 130 relative to the connecting member 171, so as to adjust the height of the lens tube holder 120, featuring a simple structure, ease to operate, and improved adjustment precision of the first adjusting member 172.

Specifically, the third threaded member 173 includes, but is not limited to, a set screw, a metric screw, a stop screw, and the like. One end of the first threaded member 123 is located in the third threaded hole 174, and the other end is in snap-fit with the connecting member 171. By partially screwing the third threaded member 173 in the third threaded hole 174, the adjusting seat 131 and the lens tube holder 120 are adjustably and fixedly connected.

Referring to FIGs. 10 and 11, in some certain embodiments, the adjusting mechanism 170 includes a second adjusting member 175 arranged on the connecting member 171, and the second adjusting member 175 is connected with the base 110 and configured for adjusting the position of the lens tube holder 120 relative to the base 110 to adjust the pitch angle of the lens tube holder 120 relative to the base 110.

As such, the connecting member 171 can be driven by the second adjusting member 175 to perform pitch motion relative to the base 110, so as to drive the lens tube holder 120 to perform pitch adjustment relative to the base 110.

Specifically, by operating the second adjusting member 175, the connecting member 171 can be driven to perform a pitch adjustment in a clockwise or counterclockwise direction on the axial direction of the lens tube holder 120. The connection between the second adjusting member 175 and the connecting member 171 can improve the supporting stability of the second adjusting member 175. The pitch adjustment range of the connecting member 171 can be expanded by selecting the second adjusting members 175 having different lengths.

Referring to FIGs. 7 and 11, in some certain embodiments, the second adjusting member 175 includes a fourth threaded member 176, and the connecting member 171 is provided with a fourth threaded hole 177 extending in the axial direction of the lens tube holder 120; the fourth threaded member 176 is arranged through the connecting member 171 and abuts against the base 110.

As such, the connecting member 171 can be driven by rotating the fourth threaded member 176 to perform pitch motion relative to the base 110, so as to drive the lens tube holder 120 to perform pitch adjustment relative to the base 110, featuring a simple structure, ease to operate, and improved adjustment precision of the second adjusting member 175.

Specifically, the numbers of the fourth threaded members 176 and the fourth threaded holes 177 may be three. The three threaded holes are arranged in a right triangle in the circumferential direction of the connecting member 171. The fourth threaded member 176 includes, but is not limited to, a set screw, a metric screw, a stop screw, and the like. The fourth threaded hole 177 may pass through the connecting member 171 in the thickness direction of the connecting member 171. The thickness direction of the connecting member 171 is consistent with the axial direction of the lens tube holder 120. The fourth threaded member 176 is arranged through the fourth threaded hole 177. One end of the fourth threaded member 176 abuts against the base 110. By screwing the fourth threaded member 176 in the fourth threaded hole 177, the connecting member 171 and the base 110 are adjustably connected.

Referring to FIGs. 7 and 10, in some certain embodiments, the connecting member 171 is provided with a third locking mechanism 178, and the third locking mechanism 178 is configured for forming a fitting connection with the base 110 to lock the connecting member 171 on the base 110, so as to keep the lens tube holder 120 stable relative to the base 110.

As such, the connecting member 171 can be fixed on the base 110 through the third locking mechanism 178, thus avoiding the movement of the connecting member 171 relative to the base 110 and ensuring the stability of the imaging system 100.

Referring to FIGs. 7 and 10, in some certain embodiments, the third locking mechanism 178 includes a third locking member 179; the third locking member 179 is arranged through the connecting member 171 and locks the connecting member 171 when the third locking member 179 is connected with the base 110.

As such, the connecting member 171 can be fixed on the base 110 through the third locking mechanism 178, thus avoiding the movement of the connecting member 171 relative to the base 110 and ensuring the stability of the imaging system 100.

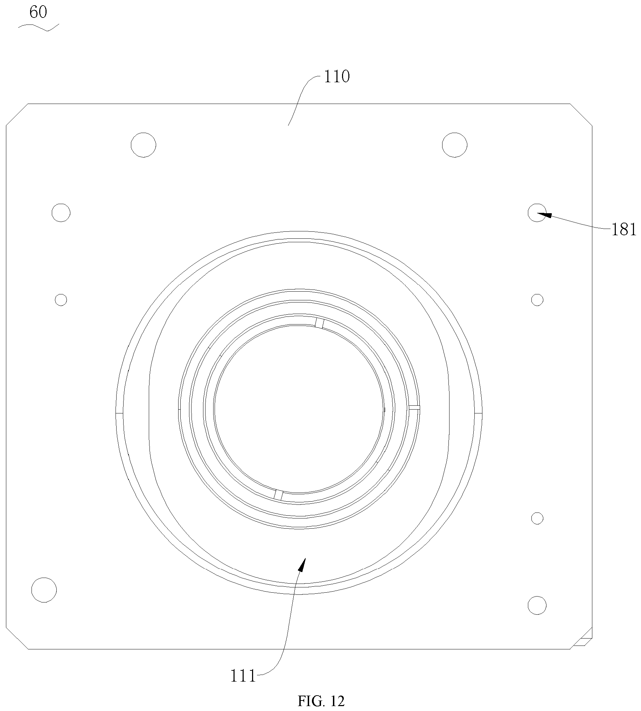

Referring to FIGs. 7, 11 and 12, in some certain embodiments, the third locking member 179 includes a fifth threaded member 180, and the base 110 is provided with a fifth threaded hole 181 extending in the axial direction of the lens tube holder 120; the fifth threaded member 180 is partially screwed in the fifth threaded hole 181.

As such, the connecting member 171 can be fixed on the base 110 through the fifth threaded member 180, thus avoiding the movement of the connecting member 171 relative to the base 110 and ensuring the stability of the imaging system 100. In addition, the first threaded member 123 makes it convenient to assemble and disassemble the connecting member 171 and the base 110, facilitating the adjustment of the connecting member 171.

Specifically, the numbers of the fifth threaded members 180 and the fifth threaded holes 181 may be three. The three threaded holes are arranged in a right triangle in the circumferential direction of the base 110. The fifth threaded member 180 includes, but is not limited to, a set screw, a metric screw, a stop screw, and the like. The fifth threaded hole 181 may pass through the connecting member 171 in the thickness direction of the base 110. The fifth threaded member 180 is arranged through the connecting member 171. One end of the fifth threaded member 180 is screwed in the fifth threaded hole 181. By screwing the fifth threaded member 180 in the fifth threaded hole 181, the connecting member 171 and the base 110 are detachably and fixedly connected.

Referring to FIGs. 7 and 10, the third locking member 179 further includes an elastic member 182. The elastic member 182 separately abuts against the connecting member 171 and the fifth threaded member 180. When the connecting member 171 is driven to move away from the base 110 by the second adjusting member 175, the elastic member 182 can provide a reaction force to the connecting member 171 toward the base 110 to improve the adjustment stability and the adjustment precision of the connecting member 171.

Referring to FIG. 3, the sequencing system 200 according to embodiments of the present application includes the imaging system 100.

The sequencing system 200 according to embodiments of the present application possesses a compact structure and a high sequencing efficiency.

Referring to FIG. 4, in some certain embodiments, the sequencing system 200 includes a mounting bracket 210 and a second carrier stage 220; the second carrier stage 220 is arranged on the mounting bracket 210, located in the optical axis extending direction of the lens module 20 and perpendicular to the optical axis of the lens module 20, and configured for carrying the sample of interest 300.

As such, the sample of interest 300 can be placed on the second carrier stage 220, which facilitates the imaging of the sample of interest 300 by the imaging system 100.

Specifically, the mounting bracket 210 is configured for carrying the second carrier stage 220 and the imaging system 100. The mounting bracket 210 may be a gantry frame and may be made of metal, alloy, or the like. The second carrier stage 220 may have a square plate structure. The second carrier stage 220 is arranged under the first carrier stage 10 and may move relative to the lens module 20.

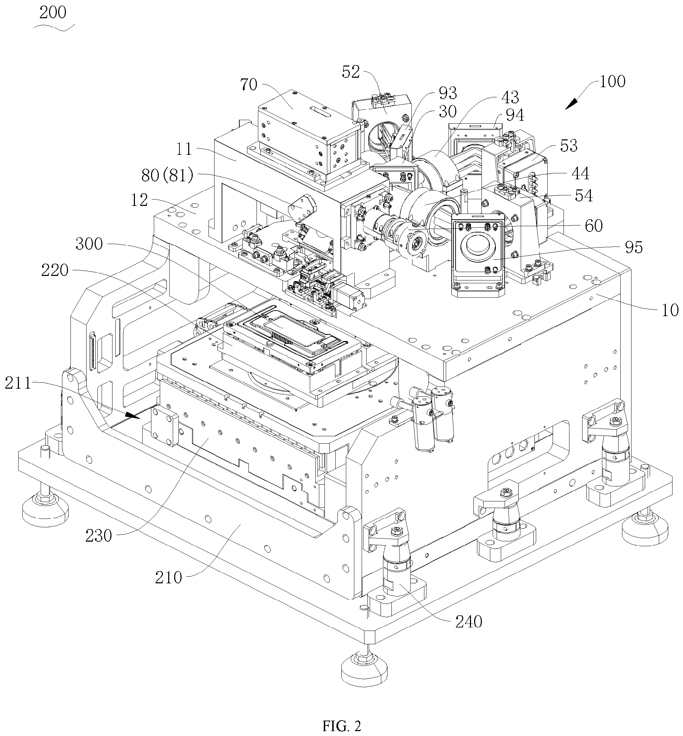

Referring to FIG. 2, in some certain embodiments, the mounting bracket 210 is formed with an accommodating space 211, and the second carrier stage 220 is accommodated in the accommodating space 211.

As such, the second carrier stage 220 can be accommodated in the mounting bracket 210 through the accommodating space 211, which facilitates the imaging of the sample of interest 300 by the imaging system 100.

Specifically, the mounting bracket 210 may be formed of a plurality of plate structures. The first carrier stage 10 is arranged on the top surface of the mounting bracket 210, and the lens module 20 is partially arranged in the accommodating space 211 through the first carrier stage 10, such that the first light beam 62 can irradiate the sample of interest 300 through the lens module 20.

Referring to FIG. 2, in some certain embodiments, the sequencing system 200 includes a movable platform mechanism 230, the movable platform mechanism 230 is accommodated in the accommodating space 211, the second carrier stage 220 is mounted on the movable platform mechanism 230, and the movable platform mechanism 230 can drive the second carrier stage 220 to move.

As such, the movable platform mechanism 230 drives the sample of interest 300 to move through driving the second carrier stage 220 to move, which facilitates the imaging of the different regions of the sample of interest 300 by the imaging system 100.

Specifically, the movable platform mechanism 230 can drive the second carrier stage 220 to move in a plane perpendicular to the optical axis of the lens module 20. The movable platform mechanism 230 may be electric, hydraulic, or pneumatic.

Referring to FIGs. 2 and 3, in some certain embodiments, the mounting bracket 210 is provided with a vibration damping structure 240 configured for reducing the vibration of the sequencing system 200.

As such, the vibration damping structure 240 can reduce the vibration of the sequencing system 200, and thus further improve the detection accuracy of the sequencing system 200.

Specifically, the vibration damping structure 240 includes, but is not limited to, a spring damper and a rubber damper. The number of the vibration damping structures 240 may be more than one. In one example, the number of the vibration damping structures 240 is six, and the six vibration damping structures 240 are distributed in mirror images on two outer sides of the mounting bracket 210 to reduce the influence of the external environment on the sequencing system 200.

In the description of this specification, the description of the terms "one embodiment", "some embodiments", "schematic embodiments", "examples", "specific examples", "some examples", or the like, means that the particular features, structures, materials, or characteristics described in the embodiments or examples are included in at least one embodiment or example of the present disclosure. In this specification, the schematic description of the aforementioned terms does not necessarily refer to the same embodiment or example. Moreover, the particular features, structures, materials, or characteristics described may be combined in any embodiment or example in any appropriate manner.

Although the embodiments of the present disclosure have been illustrated and described, it will be appreciated by those of ordinary skill in the art that various changes, modifications, replacements, and variations can be made to these embodiments without departing from the principle and purpose of the present disclosure, and the scope of the present disclosure is defined by the claims and equivalents therefore.

The present invention may be also defined by the following items.
1. An imaging system, comprising:
   a first carrier stage comprising a benchtop;
   a lens module arranged on the first carrier stage, wherein the optical axis of the lens module is perpendicular to the benchtop;
   a first support seat arranged on the benchtop, wherein the first support seat is provided with a light source module, a focusing module, and a first splitter module; the light source module is arranged aside from the optical axis of the lens module; the focusing module and the first splitter module are located on the optical axis of the lens module; the first splitter module is located between the lens module and the focusing module; and
   a second splitter module, a focusing lens set, and a detector module that are arranged on the benchtop, wherein at least part of the second splitter module is located on the optical axis of the lens module and located between the first splitter module and the lens module; the focusing lens set is located between the second splitter module and the detector module.
2. The imaging system according to item 1, wherein
   an optical correction element is arranged between the lens module and the second splitter module, and the optical correction element can be moved into or out of the optical path of the imaging system;
   when the optical correction element is moved into the imaging optical path, the optical correction element is located on the optical axis of the lens module.
3. The imaging system according to item 2, wherein
   the lens module comprises an objective lens, and the center deviation between the optical correction element and the objective lens is not greater than 0.5 mm.
4. The imaging system according to item 2 or 3, wherein
   the first support seat comprises a first surface and a second surface, the first surface of the first support seat is parallel to the optical axis of the lens module, and the second surface of the first support seat is perpendicular to the optical axis of the lens module;
   the light source module and the focusing module are respectively mounted on the first surface of the first support seat and the second surface of the first support seat.
5. The imaging system according to any one of items 2-4, wherein
   the light source module comprises a first light source,
   the first light source emits a first light beam, and the first light beam is sequentially reflected by the first splitter module and transmitted by the second splitter module to irradiate a sample of interest through the lens module, so as to excite the sample of interest to generate an emission light with at least one wavelength.
6. The imaging system according to item 5, wherein
   the focusing module comprises a second light source and a focusing sensor;
   the second light source emits a second light beam, and the second light beam is sequentially transmitted by
   the first splitter module and the second splitter module to irradiate the sample of interest through the lens module;
   the focusing sensor detects the second light beam reflected by the surface of the sample of interest.
7. The imaging system according to item 6, wherein
   the first splitter module comprises a first dichroic mirror; the first dichroic mirror is located on the optical axis of the lens module and reflects the first light beam and transmits the second light beam.
8. The imaging system according to item 7, wherein
   the second splitter module comprises a second dichroic mirror; the second dichroic mirror is located on the optical axis of the lens module and between the first dichroic mirror and the lens module, receives the first light beam and the second light beam from the first dichroic mirror, and transmits the first light beam and the second light beam to the lens module.
9. The imaging system according to item 8, wherein
   the second splitter module further comprises a third dichroic mirror, a fourth dichroic mirror, and a fifth dichroic mirror;
   the first light beam excites the sample of interest to generate four emission lights with different wavelengths;
   the lens module acquires the emission lights and transmits the emission lights to the second dichroic mirror;
   the second dichroic mirror reflects the emission lights to the third dichroic mirror;
   the third dichroic mirror splits the emission lights from the second dichroic mirror into a first mixed light beam and a second mixed light beam, reflects the first mixed light beam to the fourth dichroic mirror, and transmits the second mixed light beam to the fifth dichroic mirror;
   the fourth dichroic mirror transmits a portion of the first mixed light beam to form a third light beam and reflects another portion of the first mixed light beam to form a fourth light beam;
   the fifth dichroic mirror transmits a portion of the second mixed light beam to form a fifth light beam and reflects another portion of the second mixed light beam to form a sixth light beam.
10. The imaging system according to item 9, wherein
   the imaging system comprises a second support seat mounted on the benchtop, and the second dichroic mirror, the third dichroic mirror, the fourth dichroic mirror, and the fifth dichroic mirror are all arranged on the second support seat.
11. The imaging system according to item 9 or 10, wherein
   when the optical correction element is moved into the imaging optical path, the optical correction element is located between the lens module and the second dichroic mirror and on the optical axis of the lens module.
12. The imaging system according to any one of items 9-11, wherein
   the detector module comprises a first image sensor, a second image sensor, a third image sensor, and a fourth image sensor that are arranged on the benchtop;
   the first image sensor is configured for receiving the third light beam and forming a first image;
   the second image sensor is configured for receiving the fourth light beam and forming a second image;
   the third image sensor is configured for receiving the fifth light beam and forming a third image;
   the fourth image sensor is configured for receiving the sixth light beam and forming a fourth image.
13. The imaging system according to item 12, wherein
   the focusing lens set comprises a first tube lens, a second tube lens, a third tube lens, and a fourth tube lens;
   the first tube lens is arranged between the fourth dichroic mirror and the first image sensor and is configured for converging the third light beam onto the first image sensor;
   the second tube lens is arranged between the fourth dichroic mirror and the second image sensor and is configured for converging the fourth light beam onto the second image sensor;
   the third tube lens is arranged between the fifth dichroic mirror and the third image sensor and is configured for converging the fifth light beam onto the third image sensor;
   the fourth tube lens is arranged between the fifth dichroic mirror and the fourth image sensor and is configured for converging the sixth light beam onto the fourth image sensor.
14. The imaging system according to item 13, wherein
   the imaging system comprises a mirror set mounted on the benchtop; the mirror set comprises a first mirror, a second mirror, a third mirror, and a fourth mirror;
   the first mirror is arranged between the first tube lens and the first image sensor and is configured for reflecting the third light beam from the first tube lens to the first image sensor;
   the second mirror is arranged between the second tube lens and the second image sensor and is configured for reflecting the fourth light beam from the second tube lens to the second image sensor;
   the third mirror is arranged between the third tube lens and the third image sensor and is configured for reflecting the fifth light beam from the third tube lens to the third image sensor;
   the fourth mirror is arranged between the fourth tube lens and the fourth image sensor and is configured for reflecting the sixth light beam from the fourth tube lens to the fourth image sensor.
15. The imaging system according to any one of items 5-14, wherein the light source module further comprises:
   a base;
   a lens tube holder arranged on the base;
   a first optical assembly arranged on the lens tube holder; and
   a second optical assembly arranged on the lens tube holder and located on a light emergent side of the first optical assembly, wherein the second optical assembly is capable of rotating around the optical axis of the second optical assembly and moving along the optical axis of the second optical assembly relative to the lens tube holder.
16. The imaging system according to item 15, wherein
   the first optical assembly comprises an adjusting seat and a first lens set arranged in the adjusting seat, and the adjusting seat is capable of rotating around the optical axis of the first optical assembly and moving along the optical axis of the first optical assembly relative to the lens tube holder.
17. The imaging system according to item 16, wherein
   the first lens set comprises a collimating lens, and the collimating lens is configured for collimating light.
18. The imaging system according to item 16 or 17, wherein
   the first optical assembly further comprises a first mounting seat mounted in the adjusting seat, and the first lens set is mounted in the first mounting seat.
19. The imaging system according to item 18, wherein
   the first mounting seat is in a threaded connection with the adjusting seat.
20. The imaging system according to any one of items 16-19, wherein
   the first optical assembly comprises an optical fiber adapter plate arranged on the optical axis of the first lens set, and the optical fiber adapter plate is located on the side of the first lens set distal to the second optical assembly.
21. The imaging system according to any one of items 15-20, wherein
   the light source module comprises a filter element arranged in the lens tube holder, and the filter element is located between the first optical assembly and the second optical assembly.
22. The imaging system according to any one of items 16-21, wherein
   the lens tube holder is provided with a first locking mechanism, and the first locking mechanism is configured for forming a fitting connection with the adjusting seat to lock the first optical assembly on the lens tube holder.
23. The imaging system according to item 22, wherein
   the first locking mechanism comprises a first locking member, and the first locking member is arranged through the lens tube holder and locks the adjusting seat when the first locking member abuts against the adjusting seat.
24. The imaging system according to item 23, wherein
   the first locking member comprises a first threaded member, and the lens tube holder is provided with a first threaded hole extending in the radial direction of the lens tube holder; the first threaded member is partially screwed in the first threaded hole.
25. The imaging system according to any one of items 15-24, wherein
   the second optical assembly comprises a second mounting seat and a second lens set, the second lens set is mounted in the second mounting seat, and the second mounting seat is capable of rotating around the optical axis of the second optical assembly and moving along the optical axis of the second optical assembly relative to the lens tube holder.
26. The imaging system according to item 25, wherein
   the second lens set comprises at least two cylindrical lenses.
27. The imaging system according to item 25 or 26, wherein
   the lens tube holder is provided with a second locking mechanism, and the second locking mechanism is configured for forming a fitting connection with the second mounting seat to lock the second optical assembly on the lens tube holder.
28. The imaging system according to item 27, wherein
   the lens tube holder is formed with a first slot and a second slot; the first slot passes through the tube wall of the lens tube holder and extends to an end surface of the lens tube holder in the axial direction of the lens tube holder, the second slot passes through the tube wall of the lens tube holder and extends in the circumferential direction of the lens tube holder, and one end of the second slot is communicated with the first slot;
   the second locking mechanism comprises a second locking member arranged on the lens tube holder, and the second locking member is configured for adjusting the size of the first slot to adjust the inner diameter of the lens tube holder, such that the lens tube holder holds the second mounting seat to lock the second optical assembly on the lens tube holder.
29. The imaging system according to item 27 or 28, wherein
   the second locking member comprises a second threaded member, and the lens tube holder is provided with a second threaded hole extending in a tangential direction of the lens tube holder; the second threaded member is partially screwed in the second threaded hole.
30. The imaging system according to any one of items 15-29, wherein
   the imaging system further comprises an adjusting mechanism arranged on the base, the lens tube holder is connected with the adjusting mechanism, and the adjusting mechanism is configured for adjusting the position of the lens tube holder relative to the base in the radial direction of the lens tube holder and adjusting the pitch angle of the lens tube holder relative to the base.
31. The imaging system according to item 30, wherein
   the adjusting mechanism comprises a connecting member and a first adjusting member arranged on the connecting member; the connecting member is mounted on the base, and the first adjusting member is connected with the lens tube holder.
32. The imaging system according to item 31, wherein
   the first adjusting member comprises a third threaded member, and the lens tube holder is provided with a third threaded hole extending in the radial direction of the lens tube holder; the third threaded member is arranged through the connecting member and partially screwed in the third threaded hole.
33. The imaging system according to item 31 or 32, wherein
   the adjusting mechanism comprises a second adjusting member arranged on the connecting member, and the second adjusting member is connected with the base and configured for adjusting the position of the lens tube holder relative to the base to adjust the pitch angle of the lens tube holder relative to the base.
34. The imaging system according to item 33, wherein
   the second adjusting member comprises a fourth threaded member, and the connecting member is provided with a fourth threaded hole extending in the axial direction of the lens tube holder; the fourth threaded member is arranged through the connecting member and abuts against the base.
35. The imaging system according to any one of items 31-34, wherein
   the connecting member is provided with a third locking mechanism, and the third locking mechanism is configured for forming a fitting connection with the base to lock the connecting member on the base, so as to keep the lens tube holder stable relative to the base.
36. The imaging system according to item 35, wherein the third locking mechanism comprises a third locking member; the third locking member is arranged through the connecting member and locks the connecting member when the third locking member is connected with the base.
37. The imaging system according to item 36, wherein
   the third locking member comprises a fifth threaded member, and the base is provided with a fifth threaded hole extending in the axial direction of the lens tube holder; the fifth threaded member is partially screwed in the fifth threaded hole.
38. A sequencing system, comprising the imaging system according to any one of items 1-37.
39. The sequencing system according to item 38, wherein
   the sequencing system comprises a mounting bracket and a second carrier stage; the second carrier stage is arranged on the mounting bracket, located in the optical axis extending direction of the lens module and perpendicular to the optical axis of the lens module, and configured for carrying the sample of interest.
40. The sequencing system according to item 39, wherein
   the mounting bracket is formed with an accommodating space, and the second carrier stage is accommodated in the accommodating space.
41. The sequencing system according to item 40, wherein
   the sequencing system comprises a movable platform mechanism, the movable platform mechanism is accommodated in the accommodating space, the second carrier stage is mounted on the movable platform mechanism, and the movable platform mechanism can drive the second carrier stage to move.
42. The sequencing system according to any one of items 39-41, wherein
   the mounting bracket is provided with a vibration damping structure configured for reducing the vibration of the sequencing system.

## Claims

1. An imaging system, comprising:
a first carrier stage comprising a benchtop;
a lens module arranged on the first carrier stage, wherein the optical axis of the lens module is perpendicular to the benchtop;
a first support seat arranged on the benchtop, wherein the first support seat is provided with a light source module, a focusing module, and a first splitter module; the light source module is arranged aside from the optical axis of the lens module; the focusing module and the first splitter module are located on the optical axis of the lens module; the first splitter module is located between the lens module and the focusing module; and
a second splitter module, a focusing lens set, and a detector module that are arranged on the benchtop, wherein at least part of the second splitter module is located on the optical axis of the lens module and located between the first splitter module and the lens module; the focusing lens set is located between the second splitter module and the detector module.

2. The imaging system according to claim 1, wherein
an optical correction element is arranged between the lens module and the second splitter module, and the optical correction element can be moved into or out of the optical path of the imaging system;
when the optical correction element is moved into the imaging optical path, the optical correction element is located on the optical axis of the lens module;
the lens module comprises an objective lens, and the center deviation between the optical correction element and the objective lens is not greater than 0.5 mm.

3. The imaging system according to claim 1 or 2, wherein
the first support seat comprises a first surface and a second surface, the first surface of the first support seat is parallel to the optical axis of the lens module, and the second surface of the first support seat is perpendicular to the optical axis of the lens module;
the light source module and the focusing module are respectively mounted on the first surface of the first support seat and the second surface of the first support seat;
the light source module comprises a first light source, the first light source emits a first light beam, and the first light beam is sequentially reflected by the first splitter module and transmitted by the second splitter module to irradiate a sample of interest through the lens module, so as to excite the sample of interest to generate an emission light with at least one wavelength.

4. The imaging system according to claim 3, wherein
the focusing module comprises a second light source and a focusing sensor;
the second light source emits a second light beam, and the second light beam is sequentially transmitted by the first splitter module and the second splitter module to irradiate the sample of interest through the lens module;
the focusing sensor detects the second light beam reflected by the surface of the sample of interest.

5. The imaging system according to claim 4, wherein
the first splitter module comprises a first dichroic mirror; the first dichroic mirror is located on the optical axis of the lens module and reflects the first light beam and transmits the second light beam;
the second splitter module comprises a second dichroic mirror; the second dichroic mirror is located on the optical axis of the lens module and between the first dichroic mirror and the lens module, receives the first light beam and the second light beam from the first dichroic mirror, and transmits the first light beam and the second light beam to the lens module.

6. The imaging system according to claim 5, wherein
the second splitter module further comprises a third dichroic mirror, a fourth dichroic mirror, and a fifth dichroic mirror;
the first light beam excites the sample of interest to generate four emission lights with different wavelengths; the lens module acquires the emission lights and transmits the emission lights to the second dichroic mirror; the second dichroic mirror reflects the emission lights to the third dichroic mirror;
the third dichroic mirror splits the emission lights from the second dichroic mirror into a first mixed light beam and a second mixed light beam, reflects the first mixed light beam to the fourth dichroic mirror, and transmits the second mixed light beam to the fifth dichroic mirror;
the fourth dichroic mirror transmits a portion of the first mixed light beam to form a third light beam and reflects another portion of the first mixed light beam to form a fourth light beam;
the fifth dichroic mirror transmits a portion of the second mixed light beam to form a fifth light beam and reflects another portion of the second mixed light beam to form a sixth light beam.
the imaging system comprises a second support seat mounted on the benchtop, and the second dichroic mirror, the third dichroic mirror, the fourth dichroic mirror, and the fifth dichroic mirror are all arranged on the second support seat;
the detector module comprises a first image sensor, a second image sensor, a third image sensor, and a fourth image sensor that are arranged on the benchtop;
the first image sensor is configured for receiving the third light beam and forming a first image;
the second image sensor is configured for receiving the fourth light beam and forming a second image;
the third image sensor is configured for receiving the fifth light beam and forming a third image;
the fourth image sensor is configured for receiving the sixth light beam and forming a fourth image; optionally, the focusing lens set comprises a first tube lens, a second tube lens, a third tube lens, and a fourth tube lens;
the first tube lens is arranged between the fourth dichroic mirror and the first image sensor and is configured for converging the third light beam onto the first image sensor;
the second tube lens is arranged between the fourth dichroic mirror and the second image sensor and is configured for converging the fourth light beam onto the second image sensor;
the third tube lens is arranged between the fifth dichroic mirror and the third image sensor and is configured for converging the fifth light beam onto the third image sensor;
the fourth tube lens is arranged between the fifth dichroic mirror and the fourth image sensor and is configured for converging the sixth light beam onto the fourth image sensor;
optionally, the imaging system comprises a mirror set mounted on the benchtop; the mirror set comprises a first mirror, a second mirror, a third mirror, and a fourth mirror;
the first mirror is arranged between the first tube lens and the first image sensor and is configured for reflecting the third light beam from the first tube lens to the first image sensor;
the second mirror is arranged between the second tube lens and the second image sensor and is configured for reflecting the fourth light beam from the second tube lens to the second image sensor;
the third mirror is arranged between the third tube lens and the third image sensor and is configured for reflecting the fifth light beam from the third tube lens to the third image sensor;
the fourth mirror is arranged between the fourth tube lens and the fourth image sensor and is configured for reflecting the sixth light beam from the fourth tube lens to the fourth image sensor.

7. The imaging system according to any one of claims 1-6, wherein the light source module further comprises:
a base;
a lens tube holder arranged on the base;
a first optical assembly arranged on the lens tube holder; and
a second optical assembly arranged on the lens tube holder and located on a light emergent side of the first optical assembly, wherein the second optical assembly is capable of rotating around the optical axis of the second optical assembly and moving along the optical axis of the second optical assembly relative to the lens tube holder.

8. The imaging system according to claim 7, wherein
the first optical assembly comprises an adjusting seat and a first lens set arranged in the adjusting seat, and the adjusting seat is capable of rotating around the optical axis of the first optical assembly and moving along the optical axis of the first optical assembly relative to the lens tube holder;
optionally, the first lens set comprises a collimating lens, and the collimating lens is configured for collimating light;
optionally, the first optical assembly further comprises a first mounting seat mounted in the adjusting seat, and the first lens set is mounted in the first mounting seat;
optionally, the first mounting seat is in a threaded connection with the adjusting seat;
optionally, the first optical assembly comprises an optical fiber adapter plate arranged on the optical axis of the first lens set, and the optical fiber adapter plate is located on the side of the first lens set distal to the second optical assembly;
optionally, the light source module comprises a filter element arranged in the lens tube holder, and the filter element is located between the first optical assembly and the second optical assembly.

9. The imaging system according to claim 8, wherein
the lens tube holder is provided with a first locking mechanism, and the first locking mechanism is configured for forming a fitting connection with the adjusting seat to lock the first optical assembly on the lens tube holder;
optionally, the first locking mechanism comprises a first locking member, and the first locking member is arranged through the lens tube holder and locks the adjusting seat when the first locking member abuts against the adjusting seat;
optionally, the first locking member comprises a first threaded member, and the lens tube holder is provided with a first threaded hole extending in the radial direction of the lens tube holder; the first threaded member is partially screwed in the first threaded hole.

10. The imaging system according to any one of claims 7-9, wherein
the second optical assembly comprises a second mounting seat and a second lens set, the second lens set is mounted in the second mounting seat, and the second mounting seat is capable of rotating around the optical axis of the second optical assembly and moving along the optical axis of the second optical assembly relative to the lens tube holder;
the lens tube holder is provided with a second locking mechanism, and the second locking mechanism is configured for forming a fitting connection with the second mounting seat to lock the second optical assembly on the lens tube holder;
optionally, the second lens set comprises at least two cylindrical lenses.

11. The imaging system according to claim 10, wherein
the lens tube holder is formed with a first slot and a second slot; the first slot passes through the tube wall of the lens tube holder and extends to an end surface of the lens tube holder in the axial direction of the lens tube holder, the second slot passes through the tube wall of the lens tube holder and extends in the circumferential direction of the lens tube holder, and one end of the second slot is communicated with the first slot;
the second locking mechanism comprises a second locking member arranged on the lens tube holder, and the second locking member is configured for adjusting the size of the first slot to adjust the inner diameter of the lens tube holder, such that the lens tube holder holds the second mounting seat to lock the second optical assembly on the lens tube holder;
optionally, the second locking member comprises a second threaded member, and the lens tube holder is provided with a second threaded hole extending in a tangential direction of the lens tube holder; the second threaded member is partially screwed in the second threaded hole.

12. The imaging system according to any one of claims 7-11, wherein
the imaging system further comprises an adjusting mechanism arranged on the base, the lens tube holder is connected with the adjusting mechanism, and the adjusting mechanism is configured for adjusting the position of the lens tube holder relative to the base in the radial direction of the lens tube holder and adjusting the pitch angle of the lens tube holder relative to the base.

13. The imaging system according to claim 12, wherein
the adjusting mechanism comprises a connecting member and a first adjusting member arranged on the connecting member; the connecting member is mounted on the base, and the first adjusting member is connected with the lens tube holder;
optionally, the first adjusting member comprises a third threaded member, and the lens tube holder is provided with a third threaded hole extending in the radial direction of the lens tube holder; the third threaded member is arranged through the connecting member and partially screwed in the third threaded hole.

14. The imaging system according to claim 13, wherein
the adjusting mechanism comprises a second adjusting member arranged on the connecting member, and the second adjusting member is connected with the base and configured for adjusting the position of the lens tube holder relative to the base to adjust the pitch angle of the lens tube holder relative to the base;
optionally, the second adjusting member comprises a fourth threaded member, and the connecting member is provided with a fourth threaded hole extending in the axial direction of the lens tube holder; the fourth threaded member is arranged through the connecting member and abuts against the base;
optionally, the connecting member is provided with a third locking mechanism, and the third locking mechanism is configured for forming a fitting connection with the base to lock the connecting member on the base, so as to keep the lens tube holder stable relative to the base;
optionally, the third locking mechanism comprises a third locking member; the third locking member is arranged through the connecting member and locks the connecting member when the third locking member is connected with the base;
optionally, the third locking member comprises a fifth threaded member, and the base is provided with a fifth threaded hole extending in the axial direction of the lens tube holder; the fifth threaded member is partially screwed in the fifth threaded hole.

15. A sequencing system, comprising the imaging system according to any one of claims 1-14.
